(19) Europäisches Patentamt
European Patent Office
Office européen des brevets

(11) EP 4 703 370 A1

(12) **EUROPEAN PATENT APPLICATION**
published in accordance with Art. 153(4) EPC

(43) Date of publication:
**04.03.2026 Bulletin 2026/10**

(21) Application number: **24795471.2**

(22) Date of filing: **23.01.2024**

(51) International Patent Classification (IPC):
***C07K 5/062*** *(2006.01)* ***C07K 5/103*** *(2006.01)*
***C07K 7/06*** *(2006.01)* ***C07K 1/06*** *(2006.01)*

(52) Cooperative Patent Classification (CPC):
**C07K 1/06; C07K 5/06017; C07K 5/1005; C07K 7/06**

(86) International application number:
**PCT/CN2024/073623**

(87) International publication number:
**WO 2024/222082 (31.10.2024 Gazette 2024/44)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **28.04.2023 CN 202310479251**
**28.04.2023 CN 202310479134**

(71) Applicant: **Shenzhen Dikeman Biotechnology Co., Ltd.**
**Shenzhen, Guangdong 518000 (CN)**

(72) Inventors:
- **YANG, Chaowen**
  **Shenzhen, Guangdong 518000 (CN)**
- **YE, Liu**
  **Shenzhen, Guangdong 518000 (CN)**
- **LIU, Liangxian**
  **Shenzhen, Guangdong 518000 (CN)**

(74) Representative: **Cabinet Chaillot**
**16/20, avenue de l'Agent Sarre**
**B.P. 74**
**92703 Colombes Cedex (FR)**

(54) **AMINO ACID-DERIVED CERAMIDE, SYNTHESIS METHOD THEREFOR AND USE THEREOF**

(57) An amino acid-derived ceramide having a structure of general Formula I, or an isomer thereof:

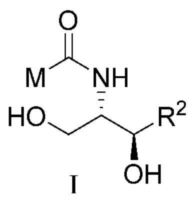

I

wherein, M is $R^3$ or

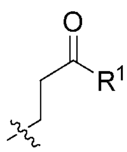

,

and $R^1$ is a residue resulting from condensation of a polypeptide; $R^3$ is selected from residues resulting from condensation of alanine, threonine, proline, asparagine, glutamine, leucine, tryptophan, serine, valine, methionine, tyrosine, histidine, L-aspartic acid-4-tert-butyl ester, L-aspartic acid-1-tert-butyl ester, glycine, isoleucine, phenylalanine, lysine, arginine, cysteine, L-glutamic acid-5-tert-butyl ester, L-glutamic acid-1-tert-butyl ester, or a polypeptide; the polypeptide is formed by condensation of 2 to 10 amino acids; $R^2$ is selected from one of the following structures: $-C_{15}H_{29}$, $-C_{15}H_{31}$, $-C_{15}H_{27}$, $-CHOHC_{14}H_{27}$, $-CHOHC_{14}H_{29}$. The compound exhibits excellent effects in moisturizing, anti-oxidation, anti-glycation, skin barrier repair, tissue healing, etc.

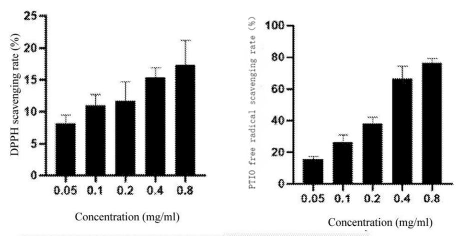


Fig. 1

EP 4 703 370 A1

## Description

### Field of the invention

The present invention belongs to the field of biomedicine, in particular relating to amino acid-derived ceramides, synthesis methods and applications thereof.

### Background

Ceramides (also known as molecular nails) naturally exist in the skin and constitute a critical component of the skin barrier (stratum corneum), with a content of up to 40-50% by weight. Ceramides are a type of sphingolipids composed of a long-chain sphingosine base and fatty acids, where the carbon chain length, degree of unsaturation, and number of hydroxyl group in the sphingosine and fatty acid moieties can all vary, thus ceramides represent a diverse class of compounds. Ceramides exhibit excellent performance in regulating skin barrier function, restoring skin moisture, and enhancing adhesion between skin keratinocytes.

If the amount of ceramide decreases, it will lead to skin xerosis, loss of the skin surface's defense function, making it easier for foreign substances to invade and cause secondary infections, thereby triggering skin rejection reaction. Specifically, invaders cause cytokines to be released from cells such as keratinocytes, Langerhans cells, and melanocytes in the epidermal layer, thus causing inflammatory phenomena, etc. Therefore, skin moisturizing is crucial for maintaining and improving the skin barrier. Compared to conventional moisturizers, physiological lipid mixtures containing ceramide compounds can promote the restoration of barrier function of the damaged skin. Clinical trial results show that they exhibit similar efficacy to moderate and above potency topical steroid preparations in improving symptoms in patients with atopic dermatitis.

Due to the importance of ceramides, a great many of cosmetic and pharmaceutical companies are researching and developing corresponding products. However, it is difficult to produce natural ceramides on a large scale, due to factors such as difficulties in extracting natural ceramides, high costs making them unsuitable for commercialization. Some companies are striving to develop ceramides with structures similar to those present in the skin and capable of providing the same functional benefits.

Therefore, considering the widespread market demand for functional ceramides, it is very necessary to construct novel ceramide compounds using naturally sourced and readily available raw materials, solve the problem of their insufficient supply, and enhance their efficacy.

### Summary of the Invention

An object of the present invention is to provide a structurally novel amino acid-derived ceramide, produced by coupling an amino acid or a polypeptide with a sphingosine base compound or a succinic acid-linked sphingosine base compound.

Another object of the present invention is to provide a method for synthesizing an amino acid-derived ceramide, utilizing amino acids, polypeptides, and sphingosine base compounds, or succinic acid-linked sphingosine base compounds, as raw materials.

Further object of the present invention is to provide applications of the amino acid-derived ceramide.

One of the above objects is achieved by the following technical solutions.

An amino acid-derived ceramide having a structure of general Formula I or an isomer of general Formula I:

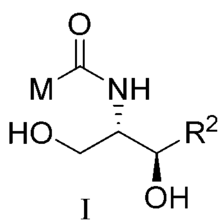

I

wherein, M is $R^3$ or

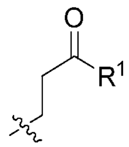,

and $R^1$ is a residue resulting from condensation of a polypeptide;

$R^3$ is selected from a residue resulting from condensation of alanine, threonine, proline, asparagine, glutamine, leucine, tryptophan, serine, valine, methionine, tyrosine, histidine, L-aspartic acid-4-tert-butyl ester, L-aspartic acid-1-tert-butyl ester, glycine, isoleucine, phenylalanine, lysine, arginine, cysteine, L-glutamic acid-5-tert-butyl ester, L-glutamic acid-1-tert-butyl ester, or a polypeptide;

the polypeptide is formed by condensation of 2 to 10 amino acids;

$R^2$ is selected from one of the following structures: $-C_{15}H_{29}$, $-C_{15}H_{31}$, $-C_{15}H_{27}$, $-CHOHC_{14}H_{27}$, $-CHOHC_{14}H_{29}$.

The residue resulting from condensation refers to the residual amino acid fragment R after condensation of the carboxyl group of a corresponding amino acid RCOOH with the amino group of a sphingosine base to form a peptide bond, for example, the residue resulting from condensation of alanine

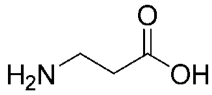

is

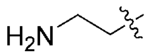,

and the residue resulting from condensation of threonine

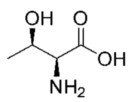 is 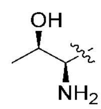;

or refers to the residual polypeptide fragment R after condensation of the carboxyl group of a corresponding polypeptide with the amino group of a sphingosine base to form a peptide bond, or after condensation of the amino group of a polypeptide with the carboxyl group of a succinic acid-linked sphingosine base

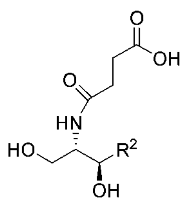

to form a peptide bond, for example, the residue resulting from condensation of diglycine

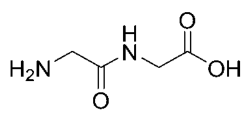

is

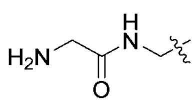,

and the residue resulting from condensation of tetrapeptide-5 $H_2N$-(β-Ala)-His-Ser-His-OH is (β-Ala)-His-Ser-His-OH.

Further, $R^3$ is selected from the residues resulting from condensation of alanine, threonine, proline, asparagine, glutamine, leucine, tryptophan, serine, valine, methionine, tyrosine, histidine, L-aspartic acid-4-tert-butyl ester, L-aspartic acid-1-tert-butyl ester, glycine, isoleucine, phenylalanine, lysine, arginine, cysteine, L-glutamic acid-5-tert-butyl ester, L-glutamic acid-1-tert-butyl ester, dipeptide, tetrapeptide, hexapeptide, octapeptide, or nonapeptide.

Further, $R^3$ is selected from the residues resulting from condensation of alanine, threonine, proline, asparagine, glutamine, leucine, tryptophan, serine, valine, methionine, tyrosine, histidine, L-aspartic acid-4-tert-butyl ester, L-aspartic acid-1-tert-butyl ester, diglycine, a snake venom peptide intermediate, alanyl-L-tyrosine, tetrapeptide-5, hexapeptide-1, hexapeptide-8, hexapeptide-9, octapeptide-3, or nonapeptide-1.

Further, $R^3$ is selected from the residues resulting from condensation of alanine, threonine, proline, asparagine, glutamine, leucine, tryptophan, serine, valine, methionine, tyrosine, histidine, L-aspartic acid-4-tert-butyl ester, L-aspartic acid-1-tert-butyl ester, diglycine, a snake venom peptide intermediate, or alanyl-L-tyrosine.

Further, $R^3$ is selected from the residues resulting from condensation of alanine, threonine, proline, asparagine, glutamine, leucine, tryptophan, serine, valine, methionine, tyrosine, histidine, L-aspartic acid-4-tert-butyl ester, L-aspartic acid-1-tert-butyl ester, glycine, isoleucine, phenylalanine, lysine, arginine, cysteine, L-glutamic acid-5-tert-butyl ester, or L-glutamic acid-1-tert-butyl ester.

Further, $R^3$ is selected from the residues resulting from condensation of alanine, threonine, proline, asparagine, glutamine, leucine, tryptophan, serine, valine, methionine, tyrosine, histidine, L-aspartic acid-4-tert-butyl ester, or L-aspartic acid-1-tert-butyl ester.

Further, $R^3$ is the residue resulting from condensation of a polypeptide.

Further, $R^3$ is selected from the residues resulting from condensation of dipeptide, tetrapeptide, hexapeptide, octapeptide, or nonapeptide.

Dipeptide is a polypeptide composed of two amino acids linked by one peptide bond; tetrapeptide is a polypeptide composed of four amino acids linked by three peptide bonds; hexapeptide is a polypeptide composed of six amino acids linked by five peptide bonds; octapeptide is a polypeptide composed of eight amino acids linked by seven peptide bonds; and nonapeptide is a polypeptide composed of nine amino acids linked by eight peptide bonds.

Further, $R^3$ is selected from the residues resulting from condensation of diglycine, a snake venom peptide intermediate, alanyl-L-tyrosine, tetrapeptide-5, hexapeptide-1, hexapeptide-8, hexapeptide-9, octapeptide-3, or nonapeptide-1.

Further, $R^3$ is selected from the residues resulting from condensation of diglycine, a snake venom peptide intermediate, or alanyl-L-tyrosine.

Further, $R^1$ is the residue resulting from condensation of a polypeptide.

Further, $R^1$ is selected from the residues resulting from condensation of dipeptide, tetrapeptide, hexapeptide, octapeptide, or nonapeptide.

Further, $R^1$ is selected from the residues resulting from condensation of diglycine, a snake venom peptide intermediate, alanyl-L-tyrosine, tetrapeptide-5, hexapeptide-1, hexapeptide-8, hexapeptide-9, octapeptide-3, or nonapeptide-1.

Further, $R^1$ is selected from the residues resulting from condensation of tetrapeptide-5, hexapeptide-1, hexapeptide-8, hexapeptide-9, octapeptide-3, or nonapeptide-1.

The amino acids constituting the polypeptide of $R^3$ or $R^1$ are selected from alanine, threonine, proline, asparagine, glutamine, leucine, tryptophan, serine, valine, methionine, tyrosine, histidine, L-aspartic acid-4-tert-butyl ester, L-aspartic acid-1-tert-butyl ester, glycine, isoleucine, phenylalanine, lysine, arginine, cysteine, L-glutamic acid-5-tert-butyl ester, and L-glutamic acid-1-tert-butyl ester.

Diglycine has a structural formula of

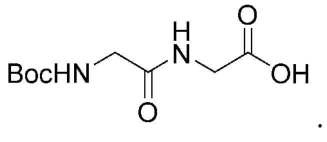

The snake venom peptide intermediate (dipeptide-1) has a structural formula of

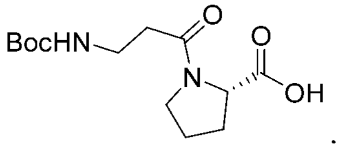

Alanyl-L-tyrosine (dipeptide-2) has a structural formula of

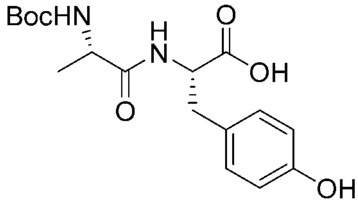
.

Tetrapeptide-5 has a structural formula of H-(b-Ala)-His-Ser-His-OH.

Hexapeptide-1 has a structural formula of H-Nle-Ala-His-(D-Phe)-Arg-Trp-$NH_2$.

Hexapeptide-8 has a structural formula of H-Glu-Glu-Met-Gln-Arg-Arg-$NH_2$.

Hexapeptide-9 has a structural formula of H-Gly-Pro-Gln-Gly-Pro-Gln-OH.

Octapeptide-3 has a structural formula of H-Glu-Glu-Met-Gln-Arg-Arg-Ala-Asp-$NH_2$.

Nonapeptide-1 has a structural formula of H-Met-Pro-D-Phe-Arg-D-Trp-Phe-Lys-Pro-Val-$NH_2$.

Further, $R^2$ is selected from one of the following structures:

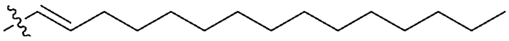

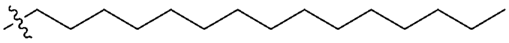

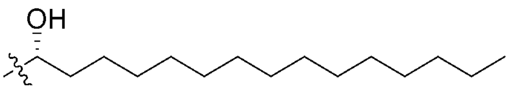

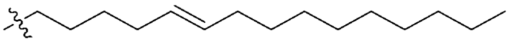

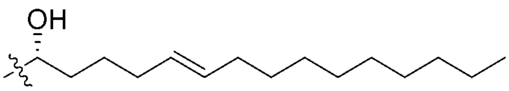

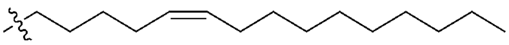

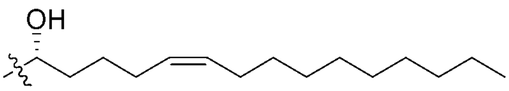

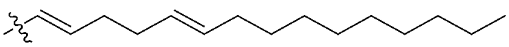

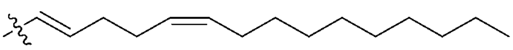
.

Further, $R^2$ is selected from one of the following structures:

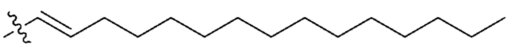

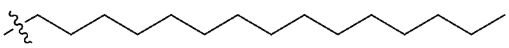

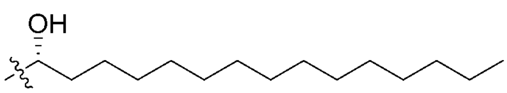
,

corresponding to sphingosine, dihydrosphingosine, and phytosphingosine, respectively.

Further, $R^3$ is the residue resulting from condensation of alanine.

Further, $R^3$ is the residue resulting from condensation of threonine.

Further, $R^3$ is the residue resulting from condensation of proline.

Further, $R^3$ is the residue resulting from condensation of asparagine.

Further, $R^3$ is the residue resulting from condensation of glutamine.

Further, $R^3$ is the residue resulting from condensation of leucine.

Further, $R^3$ is the residue resulting from condensation of tryptophan.

Further, $R^3$ is the residue resulting from condensation of serine.

Further, $R^3$ is the residue resulting from condensation of valine.

Further, $R^3$ is the residue resulting from condensation of methionine.

Further, $R^3$ is the residue resulting from condensation of tyrosine.

Further, $R^3$ is the residue resulting from condensation of histidine.

Further, $R^3$ is the residue resulting from condensation of L-aspartic acid-4-tert-butyl ester or L-aspartic acid-1-tert-butyl ester.

Further, $R^3$ is the residue resulting from condensation of diglycine.

Further, $R^3$ is the residue resulting from condensation of the snake venom peptide intermediate.

Further, $R^3$ is the residue resulting from condensation of alanyl-L-tyrosine.

Further, $R^3$ is the residue resulting from condensation of tetrapeptide-5.

Further, $R^3$ is the residue resulting from condensation of hexapeptide-1.

Further, $R^3$ is the residue resulting from condensation of hexapeptide-8.

Further, $R^3$ is the residue resulting from condensation of hexapeptide-9.

Further, $R^3$ is the residue resulting from condensation of octapeptide-3.

Further, $R^3$ is the residue resulting from condensation of nonapeptide-1.

Further, $R^1$ is the residue resulting from condensation of diglycine.

Further, $R^1$ is the residue resulting from condensation of the snake venom peptide intermediate.

Further, $R^1$ is the residue resulting from condensation of alanyl-L-tyrosine.

Further, $R^1$ is the residue resulting from condensation of tetrapeptide-5.

Further, $R^1$ is the residue resulting from condensation of hexapeptide-1.

Further, $R^1$ is the residue resulting from condensation of hexapeptide-8.

Further, $R^1$ is the residue resulting from condensation of hexapeptide-9.

Further, $R^1$ is the residue resulting from condensation of octapeptide-3.

Further, $R^1$ is the residue resulting from condensation of nonapeptide-1.

Further, the amino acid-derived ceramide is selected from one of the following compounds:

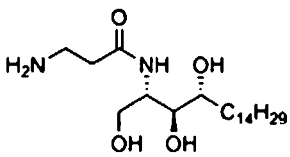
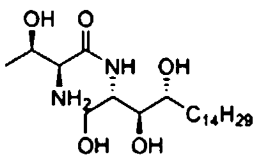
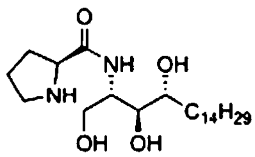
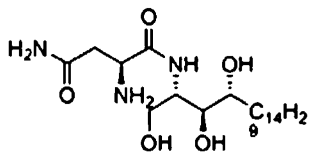

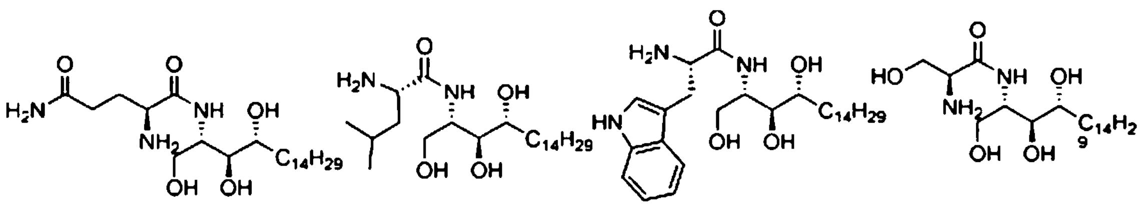

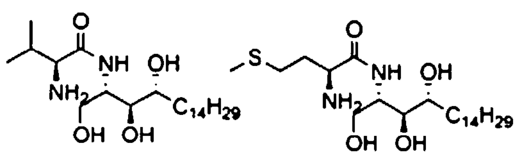
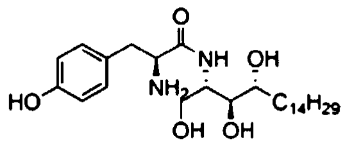
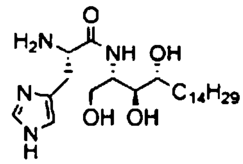

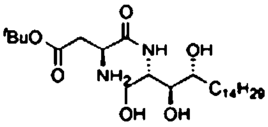
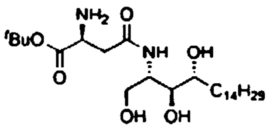
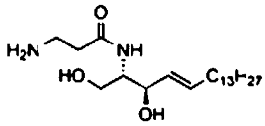
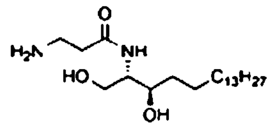

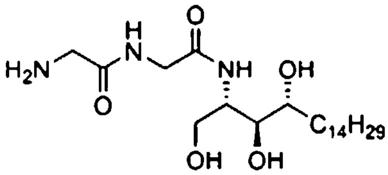
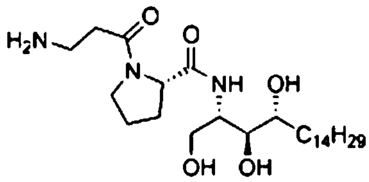
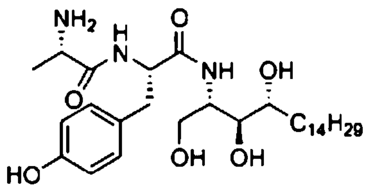

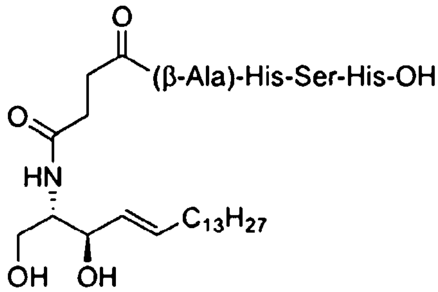
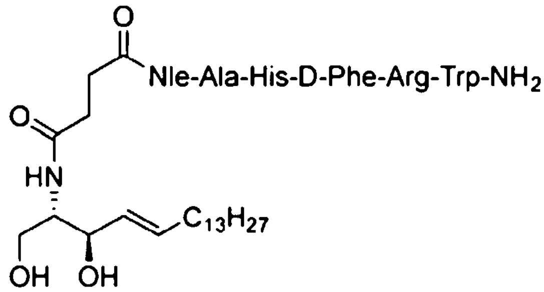

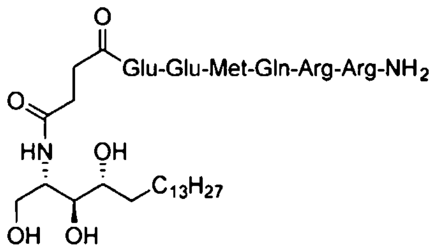
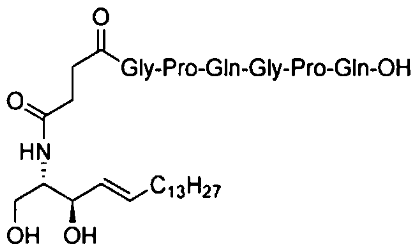

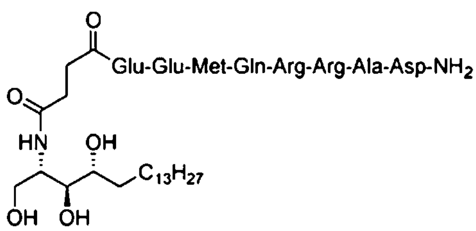
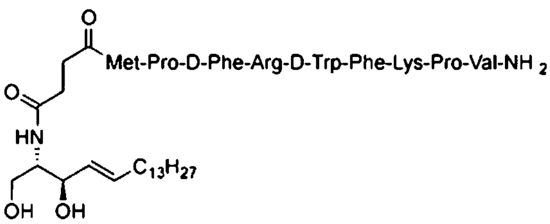

A method for synthesizing the amino acid-derived ceramide, comprising the following steps:

when M is $R^3$,
Step S1: reacting a Boc- or Fmoc-protected polypeptide or amino acid, a sphingosine base

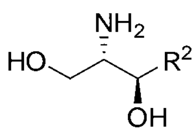
,

a condensing agent and a coupling agent to obtain compound C;
Step S2: removing the Boc or Fmoc protecting group from compound C to obtain a product; when M is

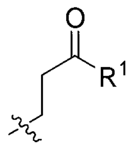

,

removing the Fmoc protecting group from the Fmoc-protected polypeptide, then reacting with a succinic acid-linked sphingosine base

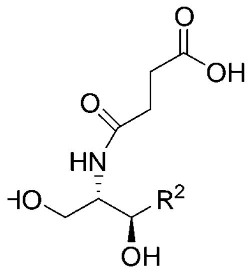

and a condensing agent to obtain a product.

When M is $R^3$, the condensing agent is EDCI or DCC, the coupling agent is N-hydroxysuccinimide, and the molar ratio of the Boc- or Fmoc-protected amino acid or polypeptide, sphingosine base, condensing agent, and coupling agent is (1 to 1.5) : 1 : (1 to 2) : (1 to 2), and the solvent for the reaction of Step S1 is dichloromethane.

When M is

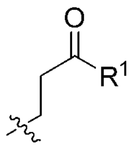

,

the condensing agents are HOBt and DIC, and the molar ratio of the polypeptide, succinic acid-linked sphingosine base

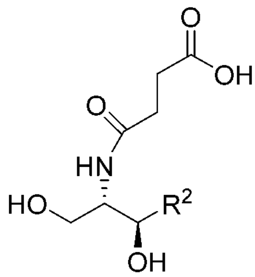

,

HOBt, and DIC is (1 to 1.5) : 1 : (1 to 2) : (1 to 2), and the solvent for the reaction is DMF.

S1 is specifically as follows: dissolving a Boc- or Fmoc-protected amino acid or polypeptide, a condensing agent (EDCI or DIC), and N-hydroxysuccinimide in dichloromethane, reacting at room temperature for a period of time, adding a sphingosine base, stirring and reacting at room temperature until TLC detects that the sphingosine base is completely consumed. Post-treatment: adding water or DCM for dilution, perfoming phase separation, filtering to remove solid, washing the organic phase with water and saturated brine respectively, extracting the aqueous phase once more with dichloromethane, combining the organic phases, drying with anhydrous sodium sulfate, filtering, evaporating under reduced pressure to obtain a crude product, and purifying by column chromatography to obtain a product.

If $R^3$ is an amino acid, S2 is specifically as follows: for the Boc protecting group, dissolving the crude product in MeOH, adding 6N hydrochloric acid slowly by dropwise, heating to 50°C and reacting until TLC detects that the crude product is completely consumed. Post-treatment: removing MeOH by a rotary evaporator, adjusting the pH value of the reaction solution to approximately 9 with saturated sodium carbonate solution, diluting with saturated brine, extracting with dichloromethane, collecting the obtained organic phase, drying with anhydrous sodium sulfate, filtering, and evaporating under reduced pressure to obtain a crude product.

For the Fmoc protecting group, dissolving the crude product in THF, adding diethylamine slowly by dropwise, reacting at room temperature until TLC detects that the crude product is completely consumed. Post-treatment: removing THF by a rotary evaporator, diluting with saturated brine, then extracting with dichloromethane, collecting the obtained organic phase, drying with anhydrous sodium sulfate, filtering, and evaporating under reduced pressure to obtain a crude product.

The method for synthesizing the amino acid-derived ceramide also comprises step S3: recrystallizing the crude product from methanol, then filtering to obtain the product; or purifying the crude product by column chromatography to obtain a product.

If $R^3$ is a polypeptide, S2 is specifically as follows: dissolving the product obtained in the previous step in DCM, adding TFA, stirring overnight at room temperature until TLC detects the raw material is completely consumed. Post-treatment: removing the reaction solution by a rotary evaporator, then adding saturated sodium carbonate solution to adjust the pH value of the reaction system to approximately 10, adding DCM followed by phase separation, washing the organic phase twice with water, back-extracting the combined aqueous phase once with DCM, combing the organic phases, drying with anhydrous sodium sulfate, filtering, and evaporating under reduced pressure to obtain a crude product, recrystallizing from methanol, and filtering to obtain a purified white solid product.

If M is

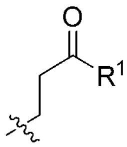

,

the specific steps are as follows: synthesizing the polypeptide on a resin, adding piperidine to remove the Fmoc protecting group from the polypeptide, weighting

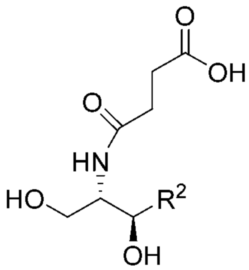

and HOBt and dissolving in DMF, adding DIC and activating under ice bath and then introducing into a reaction column, reacting at room temperature, washing the resin with DCM, DMF, and methanol, then placing the resulting crude peptide resin in a vacuum drying oven to dry. For cleavage and separation, placing the dried resin in a cleavage solution (TFA : EDT : TIPS : $H_2O$ = 92.5 : 2.5 : 2.5 : 2.5), and cleaving at room temperature, adding frozen isopropyl ether for precipitating and obtaining a white solid, vacuum-filtering and drying the white solid, then dissolving with water and acetonitrile, and separating by a semi-preparative separation instrument under a gradient of 30 : 70 (mobile phase: 1/‰ trifluoroacetic acid in water and acetonitrile) to obtain a product.

When M is

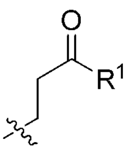

,

the synthesis method also includes the step of reacting the sphingosine base

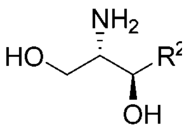

with succinic anhydride:

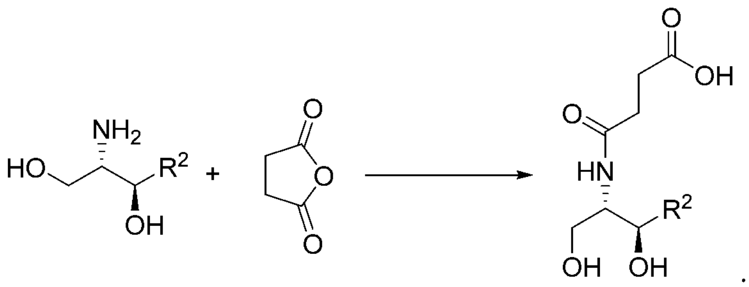

The reaction involves the addition of an organic base DIPEA, using tetrahydrofuran (THF) as the solvent. A molar ratio of the sphingosine base, succinic anhydride and DIPEA is 1: (1 to 1.5) : (1.5 to 3). The specific steps are as follows: adding succinic anhydride and sphingosine into a single-necked flask, adding THF to dissolve them, then adding DIPEA, stirring and reacting at room temperature until TLC detects the reaction is complete, extracting twice with dichloromethane and aqueous hydrochloric acid solution, extracting once with dichloromethane and saturated brine solution, combining the organic phases and purifying by column chromatography to obtain a white solid product.

The amino acid-derived ceramides exhibit at least one of the effects of skin barrier repair, tissue healing, antioxidant, anti-glycation, and moisturizing. They can be used in cosmetics, dietary supplements, pharmaceuticals, especially in cosmetic essence oils or anhydrous cosmetic formulation systems.

A composition comprising, as an active ingredient, the amino acid-derived ceramide, an isomer thereof, a pharmaceutically acceptable salt thereof, a hydrate thereof, or a solvate thereof. The composition exhibits effects of skin barrier repair, tissue healing, antioxidant, anti-glycation, or moisturizing.

The composition comprises acceptable excipients, including at least one of solubilizing agents, preservatives, antioxidants, pH-adjusting agents, penetration enhancers, liposomes, humectants, thickening agents, chelating agents, skin feel modifiers, surfactants, emulsifiers, fragrances, and colorants. The composition is in the form of creams, emulsions, solutions, films, aerosols, or sprays.

As used herein, "isomer" include tautomers and stereoisomers. Tautomers refer to structural isomers having different energies that can interconvert through low energy barriers; stereoisomers refer to compounds with the same chemical structure but different spatial arrangements of atoms or groups. Stereoisomers include enantiomers, diastereomers, conformational isomers (rotamers), geometric isomers (cis/trans isomers), and atropisomers, etc.

As used herein, "pharmaceutically acceptable salt" means a salt of one aspect of the present invention that is pharmaceutically acceptable and has the desired pharmacological activity of the parent compound. Such salts include: (1) acid addition salts formed with inorganic acids or organic acids, inorganic acids such as hydrochloric acid, hydrobromic acid, sulfuric acid, nitric acid, phosphoric acid, and the like; organic acids such as acetic acid, propionic acid, hexanoic acid, cyclopentylpropionic acid, glycolic acid, pyruvic acid, lactic acid, malonic acid, succinic acid, malic acid, maleic acid, fumaric acid, tartaric acid, citric acid, benzoic acid, 3-(4-hydroxybenzoyl)benzoic acid, cinnamic acid, mandelic acid, methanesulfonic acid, ethanesulfonic acid, 1, 2-ethanedisulfonic acid, 2-hydroxyethylsulfonic acid, benzenesulfonic acid, 4-chlorobenzenesulfonic acid, 2-naphthalenesulfonic acid, 4-toluenesulfonic acid, camphorsulfonic acid, 4-methylbicyclo [2,2,2]-oct-2-ene-1-carboxylic acid, glucoheptonic acid, 3-phenylpropionic acid, trimethylacetic acid, tert-butylacetic acid, dodecylsulfuric acid, gluconic acid, glutamic acid, hydroxynaphthoic acid, salicylic acid, stearic acid and muconic acid; or (2) salts formed when an acid proton present in the parent compound is substituted.

As used herein, "hydrate" means a compound that is bound to water. The binding between the compound and water includes non-covalent binding.

As used herein, "solvate" means a complex formed by solute molecules or ions and solvent molecules or ions.

Unless otherwise specified, the term "compound of the present invention" includes the compound itself, its pharmaceutically acceptable salts, its hydrates, its solvates, its isomers.

The present invention exhibits the following beneficial effects:

The present invention uses amino acids or polypeptides as starting materials, and constructs a class of structurally novel amino acid-derived ceramides by reacting them with sphingosine bases bearing long alkyl chains. These compounds enhance the lipid solubility of the amino acid compounds or polypeptides, facilitating skin penetration. They exhibit excellent efficacy in aspects of antioxidant, anti-glycation, skin barrier repair, tissue healing, moisturizing properties and the like, and can be used in dietary supplements, cosmetics, and pharmaceuticals. The enhanced lipid solubility of these compounds makes them more suitable for use in oil-based skincare formulation systems, solving the problem that non-modified polypeptides cannot be used in oil-based systems.

## Brief description of the drawings

Figures 1 to 12 are bar graphs showing the results of the antioxidant experiments in Example 30;

Figures 13 to 15 are bar graphs showing the results of the anti-glycation experiments in Example 31;

Figures 16 to 25 are mass spectra of the compounds in Examples 20 to 29;

Figure 26 shows the moisture absorption rate-time curves of hyaluronic acid, diglycine-phytosphingosine, snake venom peptide-phytosphingosine, and dipeptide-2-phytosphingosine at 81% ambient humidity as described in Example 32;

Figure 27 shows the moisture absorption rate-time curves of tetrapeptide-5-phytosphingosine, tetrapeptide-5-sphingosine, hexapeptide-1-phytosphingosine, and hexapeptide-1-sphingosine at 81% ambient humidity as described in Example 32;

Figure 28 shows the moisture absorption rate-time curves of hexapeptide-8-phytosphingosine, hexapeptide-9-phytosphingosine, hexapeptide-9-sphingosine, and octapeptide-3-phytosphingosine at 81% ambient humidity as described in Example 32;

Figure 29 shows the moisture absorption rate-time curves of nonapeptide-1-phytosphingosine and nonapeptide-1-sphingosine at 81% ambient humidity, and hyaluronic acid and diglycine-phytosphingosine at 43% ambient humidity as described in Example 32;

Figure 30 shows the moisture absorption rate-time curves of snake venom peptide-phytosphingosine, dipeptide-2-phytosphingosine, tetrapeptide-5-phytosphingosine, and tetrapeptide-5-sphingosine at 43% ambient humidity as described in Example 32;

Figure 31 shows the moisture absorption rate-time curves of hexapeptide-1-phytosphingosine, hexapeptide-1-sphingosine, hexapeptide-8-phytosphingosine, and hexapeptide-9-phytosphingosine at 43% ambient humidity as described in Example 32;

Figure 32 shows the moisture absorption rate-time curves of hexapeptide-9-sphingosine, octapeptide-3-phytosphingosine, nonapeptide-1-phytosphingosine, and nonapeptide-1-sphingosine at 43% ambient humidity as described in Example 32.

## Detailed description of the invention

The present invention will now be further described with reference to specific examples.

All reactions were carried out under a nitrogen atmosphere. Unless otherwise stated, chemicals were commercially available and were used without further purification. Dichloromethane, tetrahydrofuran, pyridine, and N,N-dimethylformamide used in the experiments were all anhydrous solvents. Thin layer chromatography (TLC) was performed on 60F254 silica gel plates. Silica gel column chromatography was performed on Qingdao Ocean Silica Gel (particle size of 0.040 to 0.063 mm). TLC was visualized using UV light (254 nm) or iodine. NMR spectra were characterized using a Bruker DPX 400 NMR spectrometer with $^1$H NMR at 400 MHz, and the solvents were deuterated methanol, deuterated DMSO, or deuterated tetrahydrofuran, using tetramethylsilane (TMS) as an internal standard. Chemical shifts were reported in ppm, and coupling constants were reported in Hz. In $^1$H NMR, $\delta$ represents chemical shift, s represents a singlet, d represents a doublet, t represents a triplet, q represents a quartet, and m represents a multiplet.

EDCI refers to 1-ethyl-(3-dimethylaminopropyl)carbodiimide, HOBt refers to 1-hydroxybenzotriazole, DCC refers to N'N-dicyclohexylcarbodiimide, DIC refers to N'N-diisopropylcarbodiimide, SuOH refers to N-hydroxysuccinimide, DCM refers to dichloromethane, THF refers to tetrahydrofuran, DMF refers to N,N-dimethylformamide, Boc refers to tert-butyloxycarbonyl, Fmoc refers to 9-fluorenylmethoxycarbonyl, TFA refers to trifluoroacetic acid, EA refers to ethyl acetate, MeOH refers to methanol, and DIPEA refers to N,N-diisopropylethylamine.

Method A

Condensation reaction: dissolving a Boc-protected amino acid (1.0 eq., 50 mmol), EDCI (1.5 eq., 75 mmol), and N-hydroxysuccinimide (1.5 eq., 75 mmol) in 100 mL of dichloromethane, and reacting at room temperature for 30 min; then adding phytosphingosine (1 eq., 50 mmol), stirring and reacting at room temperature until TLC detects that phytosphingosine is completely consumed.

The amount of the Boc-protected amino acid can be adjusted to 50 to 75 mmol (1 to 1.5 eq.), and the amount of EDCI and N-hydroxysuccinimide can be adjusted to 50 to 100 mmol (1 to 2 eq.), respectively. Phytosphingosine can be replaced with sphingosine or dihydrosphingosine.

Post-treatment: adding 50 mL water for dilution; perfoming phase separation; washing the organic phase once with 100 mL of water and 100 mL of saturated brine, respectively; extracting the aqueous phase once with 60 mL of dichloromethane; combining the organic phases, then drying with anhydrous sodium sulfate, filtering, and evaporating under reduced pressure to obtain a crude product.

Deprotection reaction: dissolving the crude product (1 eq., 30 mmol) in 100 mL of MeOH; adding 6N hydrochloric acid (4 eq., 120 mmol) slowly by dropwise; heating to 50°C, and reacting until TLC detects the crude starting material is completely consumed.

Post-treatment: removing MeOH by a rotary evaporator; adjusting the pH value of the reaction solution to approximately 9 with saturated sodium carbonate solution; diluting with 100 mL of saturated brine, extracting with 80 mL of dichloromethane; collecting the obtained organic phase, then drying with anhydrous sodium sulfate, filtering, and evaporating under reduced pressure to obtain a crude product.

Crude product refinement process: recrystallizing the crude product with methanol, and filtering to obtain a purified white solid product.

Method B

Condensation reaction: dissolving a Boc-protected amino acid (1.3 eq., 50 mmol) and N-hydroxysuccinimide (1.5 eq., 75 mmol) in 100 mL of dichloromethane, adding DIC (1.5 eq., 75 mmol) by dropwise at room temperature, and reacting overnight at room temperature; then adding phytosphingosine (1 eq., 50 mmol), stirring and reacting at room temperature until TLC detects that phytosphingosine is completely consumed.

The amount of the Boc-protected amino acid can be adjusted to 50 to 75 mmol (1 to 1.5 eq.), and the amount of DIC and N-hydroxysuccinimide can be adjusted to 50 to 100 mmol (1 to 2 eq.), respectively. Phytosphingosine can be replaced with sphingosine or dihydrosphingosine.

Post-treatment: adding 80 mL water for dilution; perfoming phase separation; washing the organic phase once with 80 mL of water and 80 mL of saturated brine, respectively; extracting the aqueous phase once with 80 mL of dichloromethane; combining the organic phases, then drying with anhydrous sodium sulfate, filtering, and evaporating under reduced pressure to obtain a crude product; then purifying by column chromatography to obtain a product.

Deprotection reaction: dissolving the crude product (1 eq., 30 mmol) in 100 mL of MeOH; adding 6N hydrochloric acid (4 eq., 120 mmol) slowly by dropwise; heating to 50°C, and reacting until TLC detects the crude product is completely consumed.

Post-treatment: removing MeOH by a rotary evaporator; adjusting the pH value of the reaction solution to approximately 9 with saturated sodium carbonate solution; diluting with 100 mL of saturated brine, extracting with 80 mL of dichloromethane; collecting the obtained organic phase, then drying with anhydrous sodium sulfate, filtering, and evaporating under reduced pressure to obtain a crude product.

Crude product refinement process: recrystallizing the crude product with methanol, and filtering to obtain a white solid product; slurrying the solid with dichloromethane, and filtering to obtain a purified product.

Method C

Condensation reaction: dissolving a Fmoc-protected amino acid (1.3 eq., 50 mmol) and N-hydroxysuccinimide (1.5 eq., 75 mmol) in 100 mL of dichloromethane, adding DIC (1.5 eq., 75 mmol) by dropwise at room temperature, and reacting overnight at room temperature; then adding phytosphingosine (1 eq., 50 mmol), stirring and reacting at room temperature until TLC detects that phytosphingosine is completely consumed.

The amount of the Fmoc-protected amino acid can be adjusted to 50 to 75 mmol (1 to 1.5 eq.), and the amount of DIC and N-hydroxysuccinimide can be adjusted to 50 to 100 mmol (1 to 2 eq.), respectively. Phytosphingosine can be replaced with sphingosine or dihydrosphingosine.

Post-treatment: adding 80 mL water for dilution; perfoming phase separation; washing the organic phase once with 80 mL of water and 80 mL of saturated brine, respectively; extracting the aqueous phase once with 80 mL of dichloromethane; combining the organic phases, then drying with anhydrous sodium sulfate, filtering, and evaporating

under reduced pressure to obtain a crude product; then purifying by column chromatography to obtain a product.

Deprotection reaction: dissolving the crude product (1 eq., 20 mmol) in 20 mL of THF; adding diethylamine (4 eq., 80 mmol) slowly by dropwise; and reacting at room temperature until TLC detects the crude product was completely consumed.

Post-treatment: removing THF by a rotary evaporator; diluting with 60 mL of saturated brine; extracting with 80 mL of dichloromethane; collecting the obtained organic phase, then drying with anhydrous sodium sulfate, filtering, and evaporating under reduced pressure to obtain a crude product; then purifying by column chromatography to obtain a product.

Example 1

Alanine phytosphingosine ceramide was synthesized by Method A and designated as Compound 1.

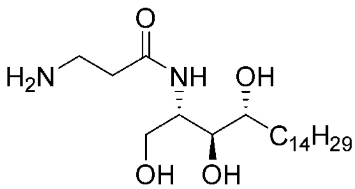

$^1$H NMR (400 MHz, Methanol-$d_4$) δ 4.14 (dt, *J* = 6.5, 4.5 Hz, 1H), 3.79 (dd, *J* = 11.2, 4.3 Hz, 1H), 3.68 (dd, *J* = 11.3, 6.5 Hz, 1H), 3.58 - 3.43 (m, 2H), 2.93 (t, *J* = 6.5 Hz, 2H), 2.48 - 2.32 (m, 2H), 1.74 - 1.62 (m, 1H), 1.57 (s, 1H), 1.29 (d, *J* = 9.3 Hz, 24H), 0.89 (t, *J* = 6.7 Hz, 3H).

Example 2

Threonine phytosphingosine ceramide was synthesized by Method B and designated as Compound 2.

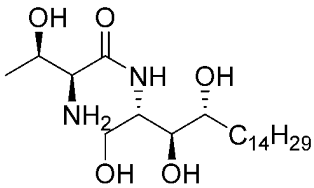

$^1$H NMR (400 MHz, Methanol-$d_4$) δ 4.15 (dt, *J* = 5.9, 4.4 Hz, 1H), 4.01 (qd, *J* = 6.4, 4.5 Hz, 1H), 3.82 - 3.68 (m, 2H), 3.57 - 3.48 (m, 2H), 3.16 (d, *J* = 4.5 Hz, 1H), 1.69 (dd, *J* = 12.3, 9.3 Hz, 1H), 1.54 (d, *J* = 10.9 Hz, 1H), 1.34 - 1.29 (s, 24H), 1.20 (d, *J* = 6.4 Hz, 3H), 0.90 (t, *J* = 6.7 Hz, 3H).

Example 3

Proline phytosphingosine ceramide was synthesized by Method A and designated as Compound 3.

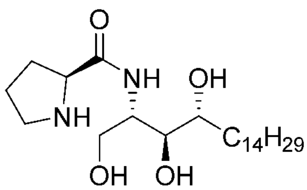

$^1$H NMR (400 MHz, Methanol-$d_4$) δ 4.09 (q, *J* = 5.1 Hz, 1H), 3.80 - 3.70 (m, 2H), 3.67 (dd, *J* = 8.7, 5.3 Hz, 1H), 3.58 - 3.44 (m, 2H), 3.02 - 2.87 (m, 2H), 2.18 - 2.05 (m, 1H), 1.86 - 1.62 (m, 4H), 1.60 - 1.45 (m, 1H), 1.30 (d, *J* = 8.6 Hz, 25H), 0.90 (t, *J* = 6.7 Hz, 3H).

Example 4

Asparagine phytosphingosine ceramide was synthesized by Method B and designated as Compound 4.

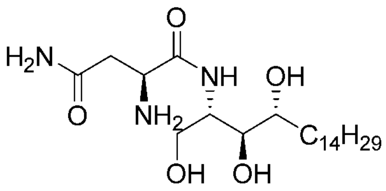

$^1H$ NMR (400 MHz, Methanol-$d_4$) δ 4.17 - 4.09 (m, 1H), 3.79 (dd, $J$ = 11.3, 4.4 Hz, 1H), 3.76 - 3.64 (m, 2H), 3.56 - 3.45 (m, 2H), 2.66 (dd, $J$ = 15.3, 5.1 Hz, 1H), 2.49 (dd, $J$ = 15.3, 7.8 Hz, 1H), 1.76 - 1.64 (m, 1H), 1.54 (d, $J$ = 11.3 Hz, 1H), 1.30 (d, $J$ = 8.5 Hz, 25H), 0.90 (t, $J$ = 6.7 Hz, 3H).

Example 5

Glutamine phytosphingosine ceramide was synthesized by Method B and designated as Compound 5.

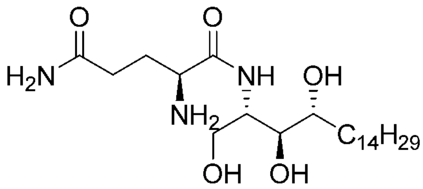

$^1H$ NMR (400 MHz, Chloroform-$d$) δ 4.65 (d, $J$ = 5.5 Hz, 1H), 4.54 (t, $J$ = 5.5 Hz, 1H), 4.37 (d, $J$ = 6.5 Hz, 1H), 4.03 (dd, $J$ = 8.5, 4.3 Hz, 1H), 3.98 - 3.84 (m, 1H), 3.52 (dq, $J$ = 10.9, 5.2 Hz, 1H), 2.30 - 1.99 (m, 3H), 1.86 (ddd, $J$ = 12.3, 9.0, 4.6 Hz, 1H), 1.55 - 1.34 (m, 2H), 1.23 - 1.18 (s, 24H), 0.85 (t, $J$ = 6.6 Hz, 3H).

Example 6

Leucine phytosphingosine ceramide was synthesized by Method A and designated as Compound 6.

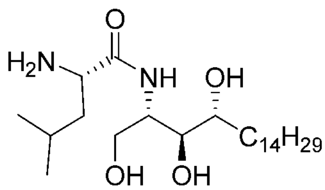

$^1H$ NMR (400 MHz, Methanol-$d_4$) δ 4.08 (td, $J$ = 5.8, 4.2 Hz, 1H), 3.81 - 3.68 (m, 2H), 3.56 (t, $J$ = 5.9 Hz, 1H), 3.51 (ddd, $J$ = 8.7, 5.9, 2.5 Hz, 1H), 3.34 (tt, $J$ = 6.3, 3.1 Hz, 1H), 1.75 - 1.49 (m, 4H), 1.42 - 1.20 (m, 25H), 0.98 - 0.91 (m, 6H), 0.91 - 0.86 (m, 3H).

Example 7

Tryptophan phytosphingosine ceramide was synthesized by Method A and designated as Compound 7.

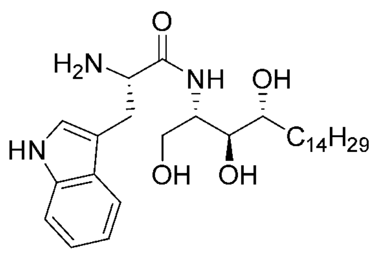

$^1H$ NMR (400 MHz, Methanol-$d_4$) δ 7.62 (d, $J$ = 7.9 Hz, 1H), 7.34 (d, $J$ = 8.0 Hz, 1H), 7.12 (s, 1H), 7.09 (ddd, J = 8.2, 6.9, 1.2 Hz, 1H), 7.04 - 6.97 (m, 1H), 4.09 (q, $J$ = 5.1 Hz, 1H), 3.72 - 3.61 (m, 3H), 3.44 (ddd, $J$ = 9.0, 6.3, 2.5 Hz, 1H), 3.39 (dd, $J$ = 6.4, 5.0 Hz, 1H), 3.22 (dd, $J$ = 14.2, 5.6 Hz, 1H), 2.95 (dd, $J$ = 14.2, 7.8 Hz, 1H), 1.63 (ddt, $J$ = 12.4, 10.2, 2.4 Hz, 1H), 1.51 (q, $J$ = 7.7 Hz, 1H), 1.29 (d, $J$ = 10.1 Hz, 24H), 0.96 - 0.83 (m, 3H).

Example 8

Serine phytosphingosine ceramide was synthesized by Method B and designated as Compound 8.

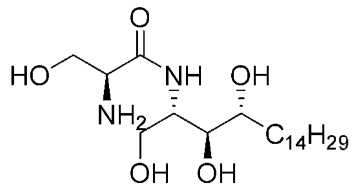

$^1H$ NMR (400 MHz, Methanol-$d_4$) δ 4.17 (q, *J* = 4.8 Hz, 1H), 3.85 - 3.66 (m, 4H), 3.59 - 3.49 (m, 2H), 3.44 (t, *J* = 5.5 Hz, 1H), 1.77 - 1.63 (m, 1H), 1.56 (d, *J* = 11.3 Hz, 1H), 1.33 - 1.30 (m, 24H), 0.92 (t, *J* = 6.7 Hz, 3H).

Example 9

Valine phytosphingosine ceramide was synthesized by Method A and designated as Compound 9.

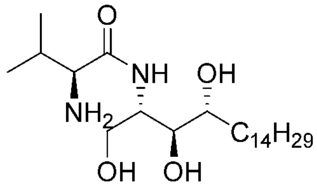

$^1H$ NMR (400 MHz, Methanol-$d_4$) δ 4.19 - 4.11 (m, 1H), 3.81 (dd, *J* = 11.3, 4.3 Hz, 1H), 3.74 (dd, *J* = 11.3, 5.9 Hz, 1H), 3.61 - 3.50 (m, 2H), 3.17 (d, *J* = 5.7 Hz, 1H), 2.01 (dq, *J* = 13.4, 6.8 Hz, 1H), 1.73-1.50 (m, 2H), 1.40-1.24 (m, 24H), 1.00 (d, *J* = 6.8 Hz, 3H), 0.96 (d, *J* = 6.9 Hz, 3H), 0.92 (t, *J* = 6.7 Hz, 3H).

Example 10

Methionine phytosphingosine ceramide was synthesized by Method B and designated as Compound 10.

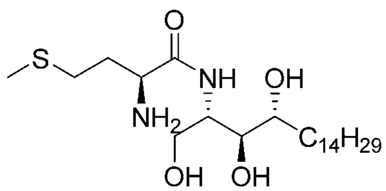

$^1H$ NMR (400 MHz, Methanol-$d_4$) δ 4.15 (td, *J* = 5.8, 4.2 Hz, 1H), 3.82 (dd, *J* = 11.3, 4.2 Hz, 1H), 3.74 (dd, *J* = 11.3, 6.1 Hz, 1H), 3.61 - 3.49 (m, 3H), 2.58 (dd, *J* = 8.6, 6.6 Hz, 2H), 2.12 (s, 3H), 2.08 - 1.97 (m, 1H), 1.86 (dt, *J* = 14.0, 7.1 Hz, 1H), 1.66 (d, *J* = 11.1 Hz, 1H), 1.57 (d, *J* = 12.1 Hz, 1H), 1.46 - 1.23 (m, 24H), 0.92 (t, *J* = 6.8 Hz, 3H).

Example 11

Tyrosine phytosphingosine ceramide was synthesized by Method B and designated as Compound 11.

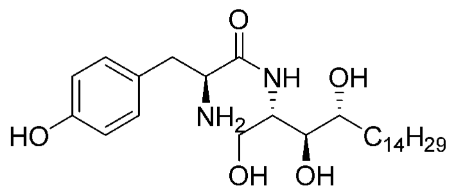

$^1H$ NMR (400 MHz, Methanol-$d_4$) δ 7.12 (d, *J* = 8.5 Hz, 2H), 6.81 - 6.74 (m, 2H), 4.17 (dt, *J* = 6.5, 4.5 Hz, 1H), 4.01 (dd, *J* = 8.3, 6.2 Hz, 1H), 3.83 (dd, *J* = 11.3, 4.3 Hz, 1H), 3.69 (dd, *J* = 11.3, 6.6 Hz, 1H), 3.49 - 3.38 (m, 2H), 3.12 (dd, *J* = 14.1, 6.3 Hz, 1H), 2.91 (dd, *J* = 14.1, 8.3 Hz, 1H), 1.72 - 1.60 (m, 1H), 1.54 (d, *J* = 10.5 Hz, 1H), 1.28 (s, 24H), 0.90 (t, *J* = 6.8 Hz,

3H).

Example 12

Histidine phytosphingosine ceramide was synthesized by Method B and designated as Compound 12.

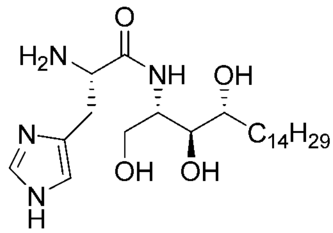

$^1H$ NMR (400 MHz, Methanol-$d_4$) δ 7.61 (s, 1H), 6.92 (s, 1H), 4.13 (dt, *J* = 6.4, 4.6 Hz, 1H), 3.79 (dd, *J* = 11.3, 4.3 Hz, 1H), 3.74 - 3.63 (m, 2H), 3.52 - 3.40 (m, 2H), 3.02 (dd, *J* = 14.7, 5.3 Hz, 1H), 2.88 (dd, *J* = 14.6, 7.4 Hz, 1H), 1.75 - 1.63 (m, 1H), 1.55 (s, 1H), 1.28 (s, 24H), 0.90 (t, *J* = 6.7 Hz, 3H).

Example 13

L-aspartic acid-4-tert-butyl ester phytosphingosine ceramide was synthesized by Method C and designated as Compound 13.

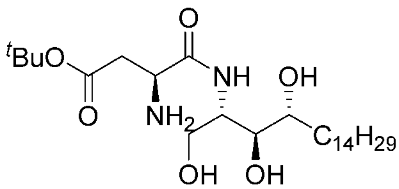

$^1H$ NMR (400 MHz, Methanol-$d_4$) δ 4.10 (q, J = 5.0 Hz, 1H), 3.75 (qd, *J* = 11.3, 5.1 Hz, 2H), 3.63 (dd, *J* = 7.2, 5.4 Hz, 1H), 3.59 - 3.47 (m, 2H), 2.70 (dd, *J* = 16.5, 5.4 Hz, 1H), 2.52 (dd, *J* = 16.5, 7.2 Hz, 1H), 1.73 - 1.60 (m, 1H), 1.54 (t, *J* = 8.0 Hz, 1H), 1.46 (s, 9H), 1.28 (s, 24H), 0.90 (t, *J* = 6.7 Hz, 3H).

Example 14

L-aspartic acid-1-tert-butyl ester phytosphingosine ceramide was synthesized by Method C and designated as Compound 14.

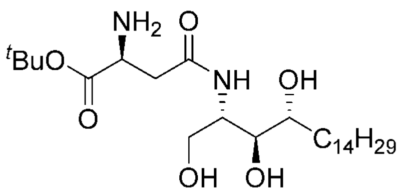

$^1H$ NMR (400 MHz, Methanol-$d_4$) δ 4.14 (dt, *J* = 6.3, 4.4 Hz, 1H), 3.79 (ddd, *J* = 9.4, 4.3, 1.7 Hz, 2H), 3.68 (dd, *J* = 11.5, 6.3 Hz, 1H), 3.57 - 3.46 (m, 2H), 2.71 (dd, *J* = 15.4, 4.3 Hz, 1H), 2.60 (dd, *J* = 15.5, 7.9 Hz, 1H), 1.71 - 1.67 (m, 1H), 1.59 - 1.51 (m, 1H), 1.48 (s, 9H), 1.36 - 1.22 (m, 24H), 0.90 (t, *J* = 6.7 Hz, 3H).

Example 15

Alanine sphingosine ceramide was synthesized by Method A and designated as Compound 15.

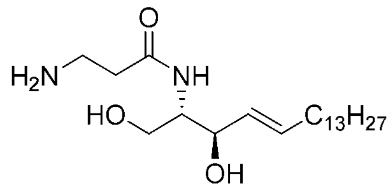

$^1$H NMR (400 MHz, Methanol-$d_4$) δ 5.70 - 5.61 (m, 1H), 5.52 - 5. 40 (m, 1H), 4.06 (t, *J* = 7.4 Hz, 1H), 3.89 - 3.82 (m, 1H), 3.68 (d, *J* = 5.0 Hz, 2H), 2.93 (t, *J* = 6.5 Hz, 2H), 2.48 - 2.32 (m, 2H), 2.15 (t, *J* = 7.6 Hz, 2H), 1.76 - 1.64 (m, 1H), 1.59 - 1.55 (m, 1H), 1.28 - 1. 22 (m, 20H), 0.91 (t, *J* = 6.8 Hz, 3H).

Example 16

Alanine dihydrosphingosine ceramide was synthesized by Method A and designated as Compound 16.

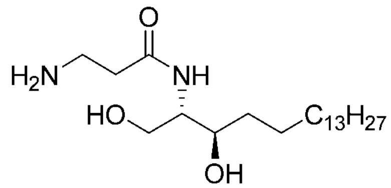

$^1$H NMR (400 MHz, Methanol-$d_4$) δ 4.12 (td, *J* = 6.1, 4.1 Hz, 1H), 3.91 - 3.81 (m, 2H), 3.79 - 3.74 (m, 1H), 2.91 (t, *J* = 6.5 Hz, 2H), 2.51 - 2.34 (m, 2H), 1.47 - 1.39 (m, 3H), 1.28 - 1.22 (m, 25H), 0.88 (t, *J* = 6.8 Hz, 3H).

Example 17

Synthesis of diglycine-phytosphingosine ceramide

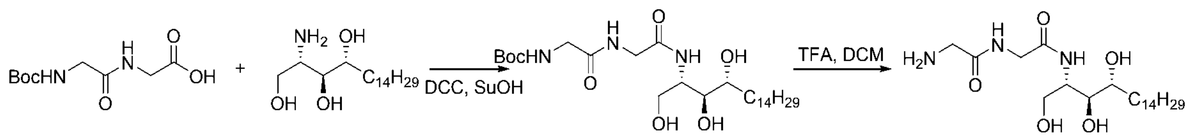


Step 1: Boc-protected diglycine (1.0 eq., 50 mmol) and N-hydroxysuccinimide (1.5 eq., 75 mmol) were dissolved in 100 mL of dichloromethane, then DCC (1.5 eq., 75 mmol) was added at room temperature and reacted overnight at room temperature. Then phytosphingosine (1 eq., 50 mmol) was added, maintaining the reaction with stirring until TLC detected that phytosphingosine was completely consumed.

Post-treatment: 80 mL of DCM was added for dilution, the mixture was filtered to remove solid. The filtrate was washed once with 80 mL of water and 80 mL of saturated brine, respectively. The aqueous phase was collected and then extracted once with 80 mL of dichloromethane. The organic phases were combined, then dried with anhydrous sodium sulfate, filtered, and evaporated under reduced pressure to obtain a crude product, which was purified by column chromatography to obtain a product.

Step 2: The product (1 eq., 30 mmol) obtained in the previous step was dissolved in 30 mL of DCM, and then 15 mL of TFA was added. The mixture was stirred and reacted overnight at room temperature until TLC detected the starting material was completely consumed.

Post-treatment: The reaction solution was evaporated, then saturated sodium carbonate solution was added to adjust the pH value of the reaction system to approximately 10. 30 mL of DCM was added followed by phase separation. The organic phase was washed twice with 30 mL of water. The combined aqueous phase was back-extracted once with 20 mL of DCM. The organic phases were combined, then dried with anhydrous sodium sulfate, filtered, and evaporated under reduced pressure to obtain a crude product, which was recrystallized with methanol and filtered to obtain a purified white solid product.

$^1$H NMR (400 MHz, Methanol-$d_4$) δ 4.15 (dt, *J* = 6.3, 4.7 Hz, 1H), 3.91 (d, *J* = 1.3 Hz, 2H), 3.78 (dd, *J* = 11.2, 4.4 Hz, 1H), 3.73 - 3.62 (m, 1H), 3.57 - 3.45 (m, 2H), 3.33 (s, 2H), 1.67 (td, *J* = 9.5, 8.7, 5.1 Hz, 1H), 1.52 (d, *J* = 14.9 Hz, 1H), 1.30 - 1.26 (m, 24H), 0.89 (t, *J* = 6.7 Hz, 3H).

Example 18

Synthesis of dipeptide-1-phytosphingosine (snake venom peptide-phytosphingosine) ceramide

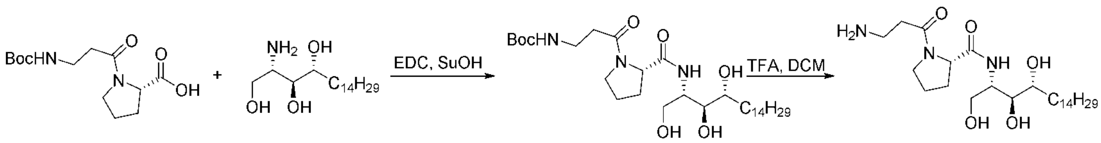


Step 1: Boc-protected dipeptide-1 (1.0 eq., 30 mmol), EDCI (1.5 eq., 45 mmol) and N-hydroxysuccinimide (1.5 eq., 45 mmol) were dissolved in 60 mL of dichloromethane, reacted at room temperature for 30 minutes. Then phytosphingosine (1 eq., 45 mmol) was added, maintaining the reaction with stirring at room temperature until TLC detected that phytosphingosine was completely consumed.

Post-treatment: 50 mL of water was added for dilution followed by phase separation. The organic phase was washed with 80 mL of water and 80 mL of saturated brine, respectively. The aqueous phase was extracted once with 60 mL of dichloromethane. The organic phases were combined, then dried with anhydrous sodium sulfate, filtered, and evaporated under reduced pressure to obtain a crude product, which was purified by column chromatography to obtain a product.

Step 2: The product (1 eq., 10 mmol) obtained in the previous step was dissolved in 10 mL of DCM, then 5 mL of TFA was added. The mixture was stirred and reacted overnight at room temperature until TLC detected the starting material was completely consumed.

Post-treatment: The reaction solution was evaporated, then saturated sodium carbonate solution was added to adjust the pH value of the reaction system to approximately 10. 30 mL of DCM was added followed by phase separation. The organic phase was washed twice with 30 mL of water. The combined aqueous phase was back-extracted once with 20 mL of DCM. The organic phases were combined, then dried with anhydrous sodium sulfate, filtered, and evaporated under reduced pressure to obtain a crude product, which was purified by column chromatography to obtain the dipeptide-1-phytosphingosine ceramide.

$^1$H NMR (400 MHz, Methanol-$d_4$) δ 4.44 (dd, *J* = 8.5, 3.9 Hz, 1H), 4.10 (td, *J* = 5.9, 4.1 Hz, 1H), 3.94 - 3.42 (m, 6H), 3.11 - 3.08 (m, 2H), 2.70 (dt, *J* = 8.7, 6.0 Hz, 2H), 2.24 (td, *J* = 8.2, 4.9 Hz, 1H), 2.15 - 1.88 (m, 3H), 1.73 - 1.50 (m, 2H), 1.32 - 1.29 (s, 24H), 0.92 (t, *J* = 6.7 Hz, 3H).

Example 19

Synthesis of dipeptide-2-phytosphingosine ceramide

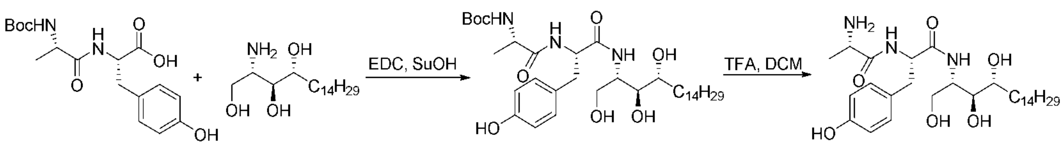


It was synthesized in accordance with the synthesis method of dipeptide-1-phytosphingosine ceramide.

$^1$H NMR (400 MHz, Chloroform-d) δ 7.07 (d, *J* = 8.3 Hz, 2H), 6.69 (d, *J* = 8.3 Hz, 2H), 4.55 (dd, *J* = 8.7, 5.9 Hz, 1H), 4.12 - 4.03 (m, 1H), 3.70 (dtd, *J* = 21.7, 10.7, 10.2, 3.6 Hz, 2H), 3.49 (ddd, *J* = 22.8, 9.6, 5.7 Hz, 3H), 3.03 (td, *J* = 14.6, 14.2, 6.4 Hz, 1H), 2.82 (dd, *J* = 13.7, 9.0 Hz, 1H), 1.67 - 1.49 (m, 2H), 1.28 - 1.24 (m, *J* = 9.8 Hz, 27H), 0.89 (t, *J* = 6.7 Hz, 3H).

Example 20

Synthesis of hexapeptide-1-sphingosine ceramide

Step 1: Synthesis using sphingosine and succinic anhydride

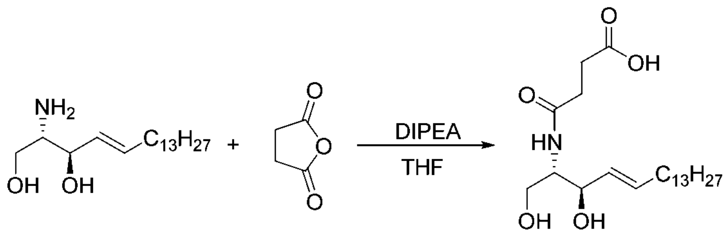


I

Succinic anhydride (3.78 g, 37.8 mmol) and sphingosine (9.5 g, 31.5 mmol) were added into a 1 L single-necked flask and dissolved in 100 mL of THF solution. DIPEA (8.2 g, 63 mmol) was added, and then the mixture was stirred and reacted at room temperature until TLC detected the reaction was complete. Extraction was performed twice with dichloromethane and aqueous hydrochloric acid, and once with dichloromethane and saturated brine. The organic phases were combined and purified by column chromatography to obtain a white solid product I (8.1 g).

Step 2: Synthesis of hexapeptide-1 sequence

1. Synthesis of MBHA Linker

7 mmol of MBHA resin (10 g, sample loading: 0.69 mmol/g) was weighted, and swelled in 100 mL of DMF for 2 h, and then drained. The mixture was washed three times with 100 mL of dry DMF, and then drained. 7.5 g of Fmoc-Linker and 2.2 g of HOBT were weighted and dissolved in 100 mL of DMF. 2.6 mL of DIC was added under ice bath. The mixture was activated in the solution for approximately 5 min, then introduced into a reaction column and reacted at room temperature for 1 h. Samples were collected for analysis. The resin was washed three times with DMF (100 mL for 3 minutes each) and drained.

2. Synthesis of Fmoc-Trp(Boc)-OH

20% piperidine/DMF solution was added for deprotection twice (100 mL for 10 min each). After completing the deprotection, the mixture was washed six times with DMF (100 mL for 3 minutes each), then drained and detected with Ninhydrin Test, K+. 8.9 g of Fmoc-Trp(Boc)-OH and 2.2 g of HOBT were weighted and dissolved in 100 mL of DMF. 2.6 mL of DIC was added under ice bath. The mixture was activated in the solution for approximately 5 minutes, then introduced into a reaction column and reacted at room temperature for 1 h. Samples were collected for analysis. The resin was washed three times with DMF (100 mL for 3 minutes each) and drained.

3. Synthesis of Fmoc-Arg(Pbf)-OH

20% piperidine/DMF solution was added for deprotection twice (100 mL for 10 min each). After completing the deprotection, the mixture was washed six times with DMF (100 mL for 3 minutes each), then drained and detected with Ninhydrin Test, K+. 9.1 g of Fmoc-Arg(Pbf)-OH and 2.2 g of HOBT were weighted and dissolved in 100 mL of DMF. 2.6 mL of DIC was added under ice bath. The mixture was activated in the solution for approximately 5 minutes, then introduced into a reaction column and reacted at room temperature for 1 hour. Samples were collected for analysis. The resin was washed three times with DMF (100 mL for 3 minutes each) and drained.

4. Synthesis of Fmoc-D-Phe-OH

20% piperidine/DMF solution was added for deprotection twice (100 mL for 10 min each). After completing the deprotection, the mixture was washed six times with DMF (100 mL for 3 minutes each), then drained. Samples were collected for analysis. 8.1 g of Fmoc-D-Phe-OH and 2.2 g of HOBT were weighted and dissolved in 100 mL of DMF. 2.6 mL of DIC was added under ice bath. The mixture was activated in the solution for approximately 5 minutes, then introduced into a reaction column and reacted at room temperature for 1 hour. Samples were collected for analysis. The resin was washed three times with DMF (100 mL for 3 minutes each) and drained.

5. Synthesis of Fmoc-His(Trt)-OH

20% piperidine/DMF solution was added for deprotection twice (100 mL for 10 min each). After completing the deprotection, the mixture was washed six times with DMF (100 mL for 3 minutes each), then drained. Samples were

collected for analysis. 12.8g of Fmoc-His(Trt)-OH and 2.2g of HOBT were weighted and dissolved in 100mL of DMF. 2.6mL of DIC was added under ice bath. The mixture was activated in the solution for approximately 5 minutes, then introduced into a reaction column and reacted at room temperature for 1 hour. Samples were collected for analysis. The resin was washed three times with DMF (100 mL for 3 minutes each) and drained.

6. Synthesis of Fmoc-Ala-OH

20% piperidine/DMF solution was added for deprotection twice (100 mL for 10 min each). After completing the deprotection, the mixture was washed six times with DMF (100 mL for 3 minutes each), then drained. Samples were collected for analysis. 5.2 g of Fmoc-Ala-OH and 2.3 g of HOBT were weighted and dissolved in 100 mL of DMF. 2.6 mL of DIC was added under ice bath. The mixture was activated in the solution for approximately 5 minutes, then introduced into a reaction column and reacted at room temperature for 1 hour. Samples were collected for analysis. The resin was washed three times with DMF (100 mL for 3 minutes each) and then drained.

7. Synthesis of Fmoc-Nle-OH

20% piperidine/DMF solution was added for deprotection twice (100 mL for 10 min each). After completing the deprotection, the mixture was washed six times with DMF (100 mL for 3 minutes each), then drained. Samples were collected for analysis. 7.4g of Fmoc-Nle-OH and 2.3g of HOBT were weighted and dissolved in 100mL of DMF. 2.6mL of DIC was added under ice bath. The mixture was activated in the solution for approximately 5 minutes, then introduced into a reaction column and reacted at room temperature for 1 hour. Samples were collected for analysis. The resin was washed three times with DMF (100 mL for 3 minutes each) and drained.

Step 3: Synthesis of hexapeptide-1-sphingosineceramide from compound I and hexapeptide-1 sequence

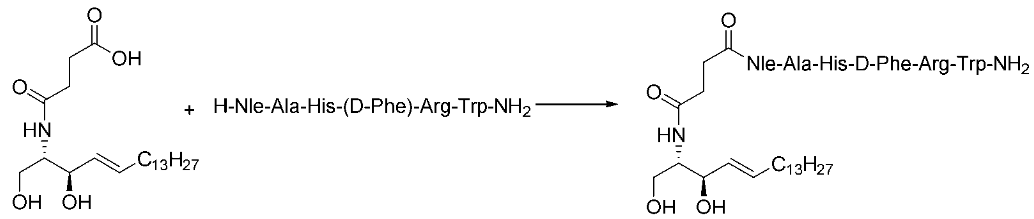


20% piperidine/DMF solution was added for deprotection twice (100 mL for 10 min each). After completing the deprotection, the mixture was washed six times with DMF (100 mL for 3 minutes each), then drained. Samples were collected for analysis. 5.8 g of compound I synthesized in Step 1 and 2.3 g of HOBt were weighed and dissolved in 100 mL of DMF. 2.6 mL of DIC was added under ice bath. The mixture was activated in the solution for approximately 5 minutes, then introduced into a reaction column and reacted at room temperature for 1 hour. Samples were collected for analysis. The resin was washed three times with DMF (100 mL for 3 minutes each) and drained. The resin was washed three times with methanol (100 mL for 3 minutes each) and drained. The obtained crude peptide resin was dried in a vacuum drying oven. The product synthesized from compound I and hexapeptide-1 sequence was named as hexapeptide-1-399.

Cleavage and preparative separation of hexapeptide-1-399: Approximately 17.8 g of the dried resin obtained after vacuum drying was weighed. 200 mL of cleavage solution (TFA : EDT : TIPS : H□O = 92.5 : 2.5 : 2.5 : 2.5) was prepared and the resin was cleaved at room temperature for 2 hours. 2 L of frozen isopropyl ether was added to cause the precipitation of a white solid. The solid was then vacuum-filtered, dried, then dissolved in water and acetonitrile. Separation was carried out using a semi-preparative separation instrument under a gradient of 30 : 70 (mobile phase: 1‰ trifluoroacetic acid in water and acetonitrile). The qualified solution was concentrated under reduced pressure and lyophilized. The mass spectrum test of the resulting compound is shown in Figure 16.

Example 21

Synthesis of hexapeptide-1-phytosphingosine ceramide

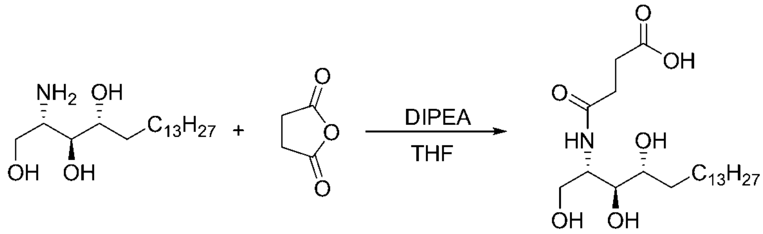


II

Step 1: Synthesis using phytosphingosine and succinic anhydride

Succinic anhydride (3.78 g, 37.8 mmol) and phytosphingosine (10 g, 31.5 mmol) were added into a 1 L single-necked flask and dissolved in 100 mL of THF solution. DIPEA (8.2 g, 63 mmol) was added, then the mixture was stirred and reacted at room temperature until TLC detected the reaction was complete. Extraction was performed twice with dichloromethane and aqueous hydrochloric acid, and once with dichloromethane and saturated brine. The organic phases were combined and purified by column chromatography to obtain a white solid product I (8.2 g).

Step 2: Synthesis of hexapeptide-1-phytosphingosine ceramide from compound II and hexapeptide-1 sequence

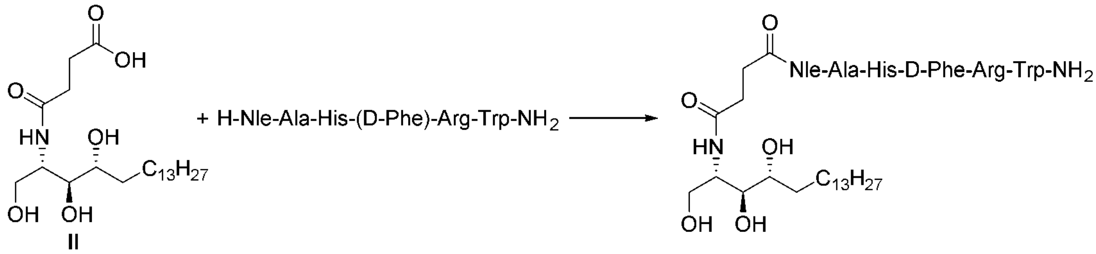


II

Reference was made to Step 3 of Example 20. The mass spectrum test of the resulting compound is shown in Figure 17.

Example 22

Synthesis of hexapeptide-8-phytosphingosine ceramide

Hexapeptide-8 sequence was synthesized in accordance with the synthesis method of hexapeptide-1.

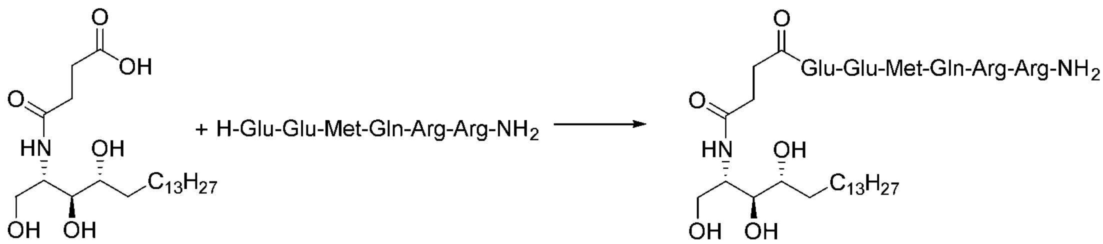


II

The synthesis was carried out in accordance with Example 21. The mass spectrum test of the resulting compound is shown in Figure 18.

Example 23

Synthesis of hexapeptide-9-sphingosine ceramide

Hexapeptide-9 sequence was synthesized in accordance with the synthesis method of hexapeptide-1.

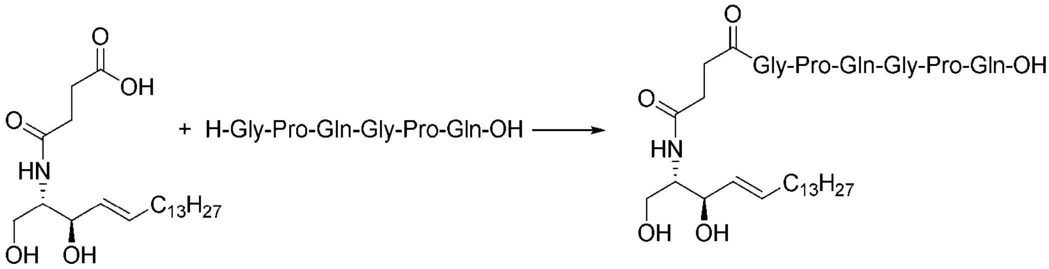


The synthesis was carried out in accordance with Example 20. The mass spectrum test of the resulting compound is shown in Figure 19.

Example 24

Synthesis of hexapeptide-9-phytosphingosine ceramide

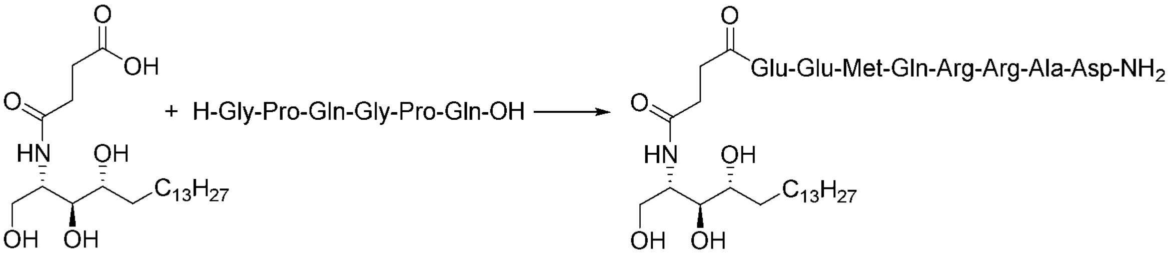


The synthesis was carried out in accordance with Example 21. The mass spectrum test of the resulting compound is shown in Figure 20.

Example 25

Synthesis of tetrapeptide-5-sphingosine ceramide

Tetrapeptide-5 sequence was synthesized in accordance with the synthesis method of hexapeptide-1.

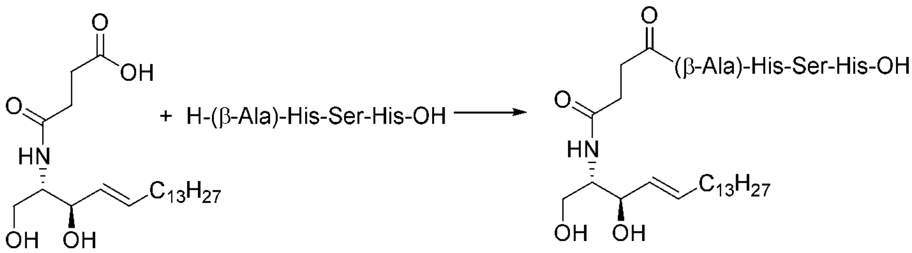


I

The synthesis was carried out in accordance with Example 20. The mass spectrum test of the resulting compound is shown in Figure 21.

Example 26

Synthesis of tetrapeptide-5-phytosphingosine ceramide

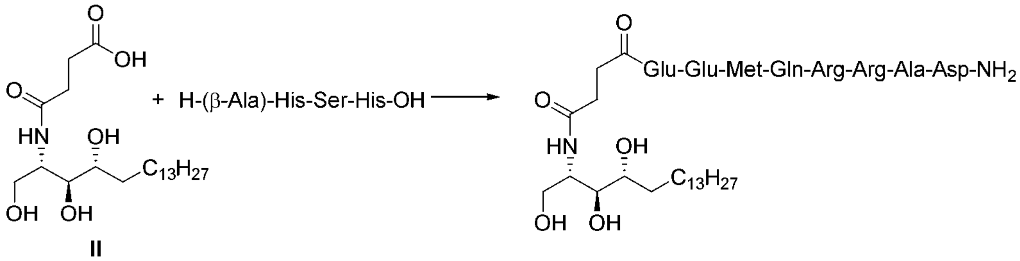


II

The synthesis was carried out in accordance with Example 21. The mass spectrum test of the resulting compound is shown in Figure 22.

Example 27

Synthesis of octapeptide-3-phytosphingosine ceramide

Octapeptide-3 sequence was synthesized in accordance with the synthesis method of hexapeptide-1.

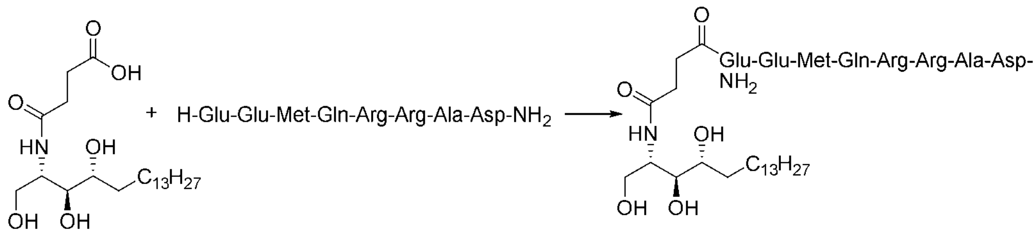


The synthesis was carried out in accordance with Example 21. The mass spectrum test of the resulting compound is shown in Figure 23.

Example 28

Synthesis of nonapeptide-1-sphingosine ceramide

Nonapeptide-1 sequence was synthesized in accordance with the synthesis method of hexapeptide-1.

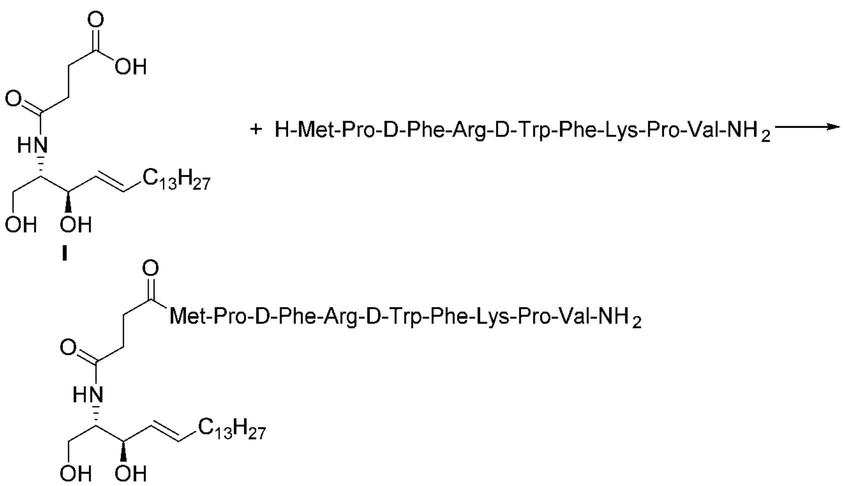


I

The synthesis was carried out in accordance with Example 20. The mass spectrum test of the resulting compound is shown in Figure 24.

Example 29

Synthesis of nonapeptide-1-phytosphingosine ceramide

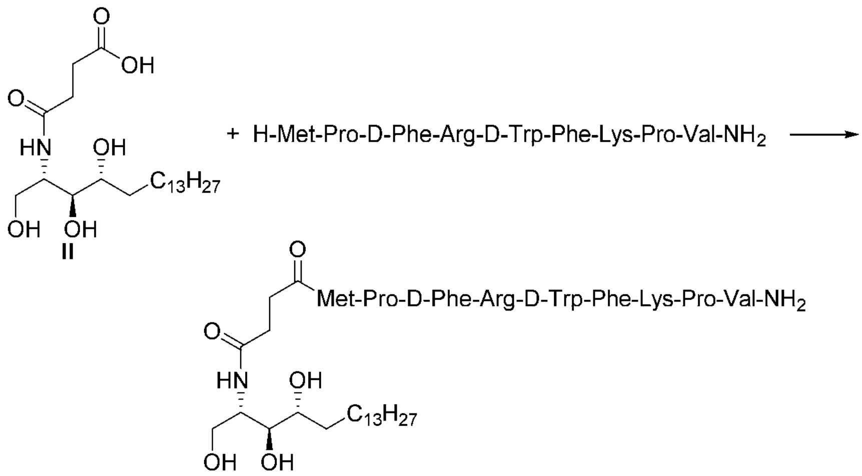


II

The synthesis was carried out in accordance with Example 21. The mass spectrum test of the resulting compound is shown in Figure 25.

Example 30

Antioxidant experiments: selecting nitrogen free radical scavenger DPPH and oxygen free radical scavenger PTIO

Determination of DPPH free radical scavenging rate

Samples of corresponding concentration were mixed with 0.1 mol/L DPPH and anhydrous ethanol solution at a volume ratio of 1:1, respectively. The blank control group was prepared by mixing DPPH and anhydrous ethanol at a volume ratio of 1:1. The mixtures were reacted at room temperature in dark for 30 minutes, and the absorbance was measured at 517 nm. The absorbance of the reaction solution of DPPH and samples of different concentration was denoted as $A_1$, the absorbance of the reaction solution of anhydrous ethanol and samples of different concentrations was denoted as $A_2$, and the absorbance of the reaction solution of DPPH and anhydrous ethanol was denoted as $A_3$. The DPPH scavenging rate of the samples was calculated as $[1-(A_1-A_2)/A_3] \times 100\%$.

Determination of PTIO free radical scavenging rate

Samples of corresponding concentration were mixed with 0.6 mmol/L PTIO and anhydrous ethanol at a volume ratio of 1:2, respectively. The blank control group was prepared by mixing PTIO and anhydrous ethanol at a volume ratio of 1:2. The mixtures were reacted at room temperature in dark for 30 minutes, and the absorbance was measured at 557 nm. The absorbance of the reaction solution of PTIO and samples of different concentration was denoted as $A_1$, the absorbance of the reaction solution of anhydrous ethanol and samples of different concentration was denoted as $A_2$, and the absorbance of the reaction solution of PTIO and anhydrous ethanol was denoted as $A_3$. The PTIO scavenging rate of the sample was calculated as $[1-(A_1-A_2)/A_3] \times 100\%$.

Figures 1 to 12 are bar graphs showing the results of the antioxidant experiments for Compounds 1, 2, 3, 4, 6, 7, 8, 9, 10, 11, 13, and 14, respectively. The amino acid ceramide compounds of the present invention exhibit excellent antioxidant properties, showing better oxygen free radical scavenging performance than that of nitrogen free radicals.

Example 31

Anti-glycation experiments

Under sterile conditions, 4 mL each of bovine serum albumin solution and glucose solution, sterilized through a sterilization membrane, were added into a sterile bottle. After mixing thoroughly, 4 mL each of sample solutions of different concentration and phosphate buffer were added. The mixture was incubated at 37°C in dark for 10 days. Aminoguanidine

was used instead of the sample as the positive control group, and phosphoric acid was used instead of the sample as the blank control group. AGEs content was determined on the 10th day of the reaction. Each group was tested in triplicate.

AGEs assay method involves taking 3 mL of glycated substance and measuring the fluorescence value (Fs) of the final glycated product at an excitation wavelength of 340 nm and an emission wavelength of 420 nm, which represents the content of total fluorescent AGEs content. The value measured with the phosphoric acid solution is donated as F0.

$$\text{Calculation of the inhibition rate (\%)} = (F0 - Fs) / F0 \times 100.$$

Figure 13 is a bar graph showing the results of the anti-glycation experiments for Compounds 1, 2, 3, and 4; Figure 14 is a bar graph showing the results of the anti-glycation experiments for Compounds 6, 7, 8, and 9; and Figure 15 is a bar graph showing the results of the anti-glycation experiments for compounds 10, 11, 13, and 14. The amino acid ceramide compounds of the present invention all exhibit excellent anti-glycation effects.

Example 32

Moisturizing performance test

Instruments: Constant temperature and humidity watertight incubator, temperature and humidity meter, sealed desiccator, volumetric flask, 1/10,000 electronic balance, drying oven.

Reagents: Hyaluronic acid (HA), compounds synthesized in Examples 1 to 13, dry micropowdered silica gel, potassium carbonate, ammonium sulfate.

Solution preparation: Saturated potassium carbonate solution (43% humidity), saturated ammonium sulfate solution (81 % humidity).

Moisture absorption rate: A weighing bottle was labeled and filled with the sample, evenly covering the entire bottom of the bottle. The weighing bottle was placed in a vacuum drying oven and dried for 1 hour. The weighing bottle was taken out and weighed on the electronic balance, the initial weight at 0 hours was recorded. The weighing bottle and saturated ammonium sulfate solution were sealed in the drying oven, and then placed in the watertight incubator (20 to 25°C). The weighing bottle was removed and weighed after 4, 8, 12, and 24 hours, respectively, recording the data. Hyaluronic acid (HA) solution is used as a control sample and tested by the same method. The saturated ammonium sulfate solution was replaced with saturated potassium carbonate solution, and the above steps were repeated.

$$\text{Calculation: Moisture absorption rate (\%)} = (M - M_0) / M_0 \times 100\%$$

M: Sample weight after moisture absorption

M0: Sample weight after drying to constant weight

Figure 26 shows the moisture absorption rate-time curves of hyaluronic acid, diglycine-phytosphingosine, snake venom peptide-phytosphingosine, and dipeptide-2-phytosphingosine (corresponding from left to right, top to bottom, the same below) at 81% ambient humidity. Figure 27 shows the moisture absorption rate-time curves of tetrapeptide-5-phytosphingosine, tetrapeptide-5-sphingosine, hexapeptide-1-phytosphingosine, and hexapeptide-1-sphingosine at 81% ambient humidity. Figure 28 shows the moisture absorption rate-time curves of hexapeptide-8-phytosphingosine, hexapeptide-9-phytosphingosine, hexapeptide-9-sphingosine, and octapeptide-3-phytosphingosine at 81% ambient humidity. Figure 29 shows the moisture absorption rate-time curves of nonapeptide-1-phytosphingosine and nonapeptide-1-sphingosine at 81% ambient humidity, and hyaluronic acid and diglycine-phytosphingosine at 43% ambient humidity. Figure 30 shows the moisture absorption rate-time curves of snake venom peptide-phytosphingosine, dipeptide-2-phytosphingosine, tetrapeptide-5-phytosphingosine, and tetrapeptide-5-sphingosine at 43% ambient humidity. Figure 31 shows the moisture absorption rate-time curves of hexapeptide-1-phytosphingosine, hexapeptide-1-sphingosine, hexapeptide-8-phytosphingosine, and hexapeptide-9-phytosphingosine at 43% ambient humidity. Figure 32 shows the moisture absorption rate-time curves of hexapeptide-9-sphingosine, octapeptide-3-phytosphingosine, nonapeptide-1-phytosphingosine, and nonapeptide-1-sphingosine at 43% ambient humidity. The ceramide compounds of the present invention exhibit good moisture absorption efficacy under both humidity environments.

The above description is merely a specific embodiment of the present invention, but the protection scope of the present invention is not limited thereto. Any changes or substitutions that can be easily conceived by any person skilled in the art within the technical scope disclosed in the present invention should be included in the scope of protection of the present invention. Therefore, the protection scope of the present invention should be subject to the protection scope of the claims.

## Claims

1. An amino acid-derived ceramide having a structure of general Formula I, or an isomer thereof:

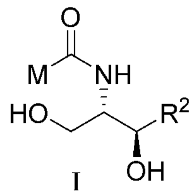

I

wherein, M is $R^3$ or

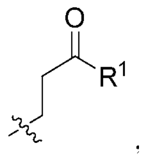

, and $R^1$ is a residue resulting from condensation of a polypeptide;
$R^3$ is selected from residues resulting from condensation of alanine, threonine, proline, asparagine, glutamine, leucine, tryptophan, serine, valine, methionine, tyrosine, histidine, L-aspartic acid-4-tert-butyl ester, L-aspartic acid-1-tert-butyl ester, glycine, isoleucine, phenylalanine, lysine, arginine, cysteine, L-glutamic acid-5-tert-butyl ester, L-glutamic acid-1-tert-butyl ester, or a polypeptide;
the polypeptide is formed by condensation of 2 to 10 amino acids;
$R^2$ is selected from one of the following structures:$-C_{15}H_{29}$, $-C_{15}H_{31}$, $-C_{15}H_{27}$, $-CHOHC_{14}H_{27}$, $-CHOHC_{14}H_{29}$.

2. The amino acid-derived ceramide according to claim 1, wherein $R^3$ is selected from the residues resulting from condensation of alanine, threonine, proline, asparagine, glutamine, leucine, tryptophan, serine, valine, methionine, tyrosine, histidine, L-aspartic acid-4-tert-butyl ester, L-aspartic acid-1-tert-butyl ester, glycine, isoleucine, phenylalanine, lysine, arginine, cysteine, L-glutamic acid-5-tert-butyl ester, L-glutamic acid-1-tert-butyl ester, dipeptide, tetrapeptide, hexapeptide, octapeptide, or nonapeptide.

3. The amino acid-derived ceramide according to claim 2, wherein $R^3$ is selected from the residues resulting from condensation of alanine, threonine, proline, asparagine, glutamine, leucine, tryptophan, serine, valine, methionine, tyrosine, histidine, L-aspartic acid-4-tert-butyl ester, L-aspartic acid-1-tert-butyl ester, diglycine, a snake venom peptide intermediate, alanyl-L-tyrosine, tetrapeptide-5, hexapeptide-1, hexapeptide-8, hexapeptide-9, octapeptide-3, or nonapeptide-1.

4. The amino acid-derived ceramide according to claim 1, wherein $R^1$ is a residue resulting from condensation of dipeptide, tetrapeptide, hexapeptide, octapeptide, or nonapeptide.

5. The amino acid-derived ceramide according to claim 4, wherein $R^1$ is a residue resulting from condensation of diglycine, a snake venom peptide intermediate, alanyl-L-tyrosine, tetrapeptide-5, hexapeptide-1, hexapeptide-8, hexapeptide-9, octapeptide-3, or nonapeptide-1.

6. The amino acid-derived ceramide according to claim 1, wherein $R^2$ is selected from one of the following structures:

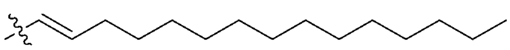

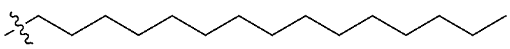

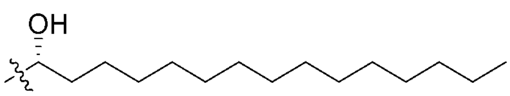

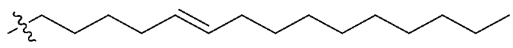

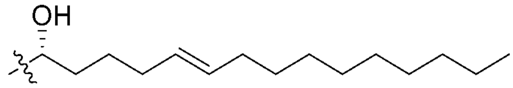

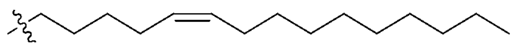

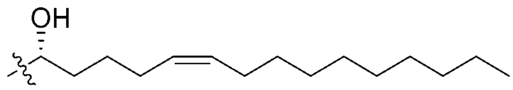

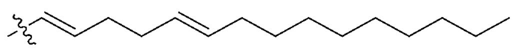

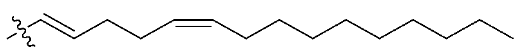

.

7. The amino acid-derived ceramide according to claim 6, wherein $R^2$ is selected from one of the following structures:

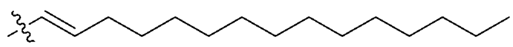

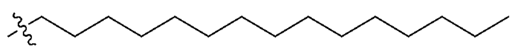

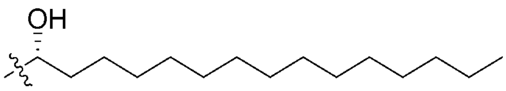

.

8. The amino acid-derived ceramide according to claim 7, **characterized in that** it is selected from one of the following compounds:

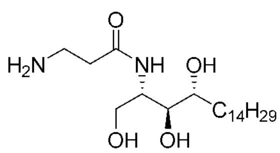

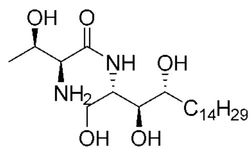

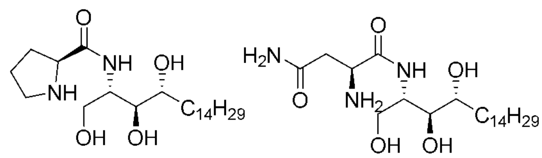

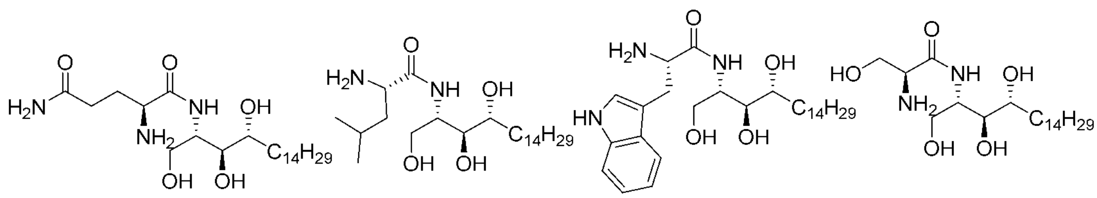

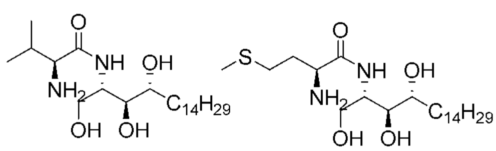

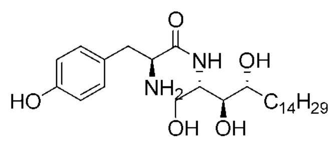

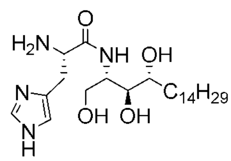

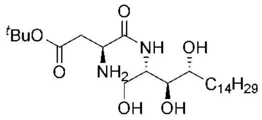
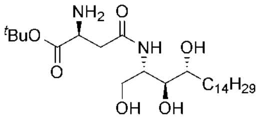
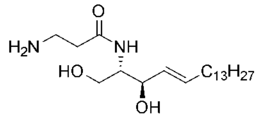
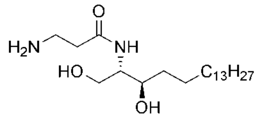

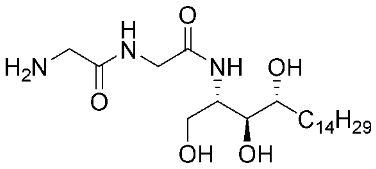
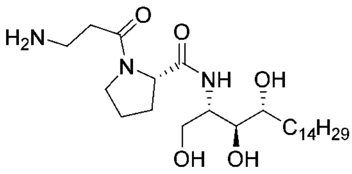
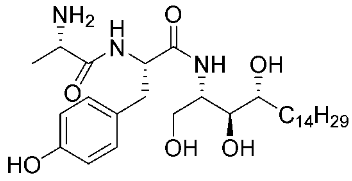

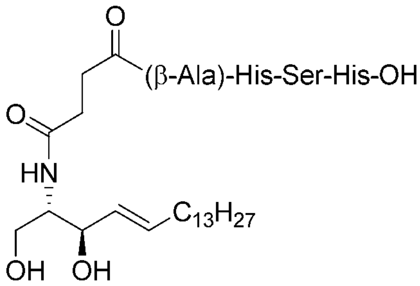
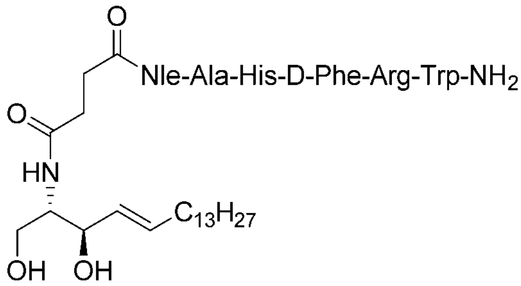

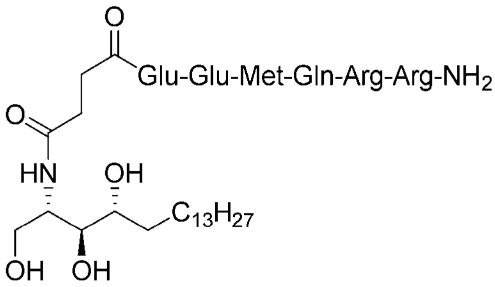
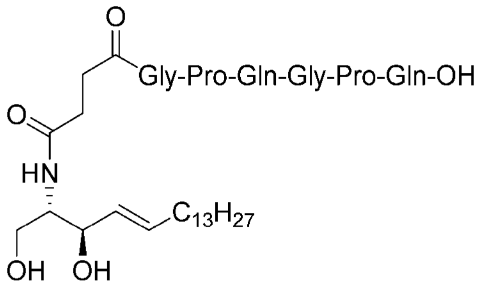

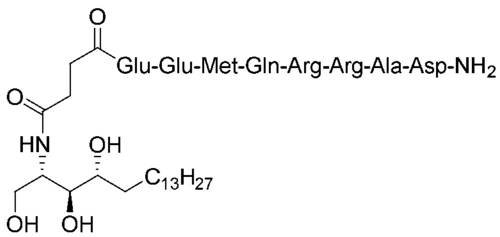
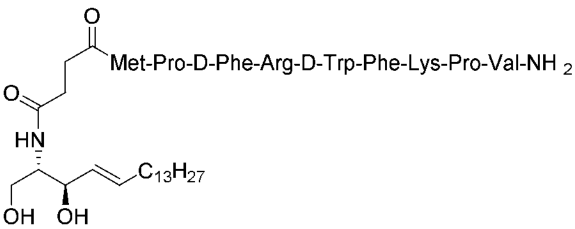
.

9. A method for synthesizing the amino acid-derived ceramide according to any one of claims 1 to 8, comprising the following steps:

when M is $R^3$,
Step S1: reacting a Boc- or Fmoc-protected polypeptide or amino acid, a sphingosine base

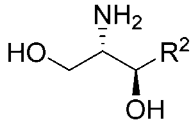
,

a condensing agent and a coupling agent to obtain compound C;
Step S2: removing the Boc or Fmoc protecting group from compound C to obtain a product; when M is

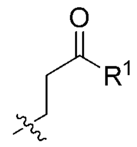

,

removing the Fmoc protecting group from a Fmoc-protected polypeptide, then reacting with a succinic acid-linked sphingosine base

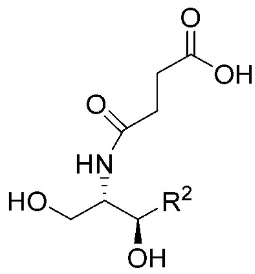

and a condensing agent to obtain a product.

**10.** The method according to claim 9, **characterized in that**, when M is $R^3$, the condensing agent is EDCI or DCC, the coupling agent is N-hydroxysuccinimide, the molar ratio of the Boc- or Fmoc-protected amino acid or polypeptide, sphingosine base, condensing agent, and coupling agent is (1 to 1.5) : 1 : (1 to 2) : (1 to 2), and the solvent for the reaction of Step S1 is dichloromethane;
when M is

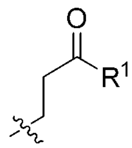

,

the condensing agents are HOBt and DIC, the molar ratio of the polypeptide, succinic acid-linked sphingosine base

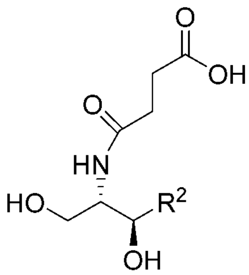

,

HOBt, and DIC is (1 to 1.5) : 1 : (1 to 2) : (1 to 2), and the solvent for the reaction is DMF.

**11.** Use of the amino acid-derived ceramide according to any one of claims 1 to 8 in cosmetics, dietary supplements, and pharmaceuticals.

**12.** The use according to claim 11, **characterized in that**, the cosmetics are cosmetic essence oils or cosmetic anhydrous formulation systems.

**13.** The use according to claim 11, **characterized in that**, the amino acid-derived ceramide provides at least one effect selected from moisturizing, skin barrier repair, tissue healing, antioxidant, and anti-glycation.

**14.** A composition comprising, as an active ingredient, the amino acid-derived ceramide according to any one of claims 1 to 8, or an isomer, a pharmaceutically acceptable salt, a hydrate, or a solvate thereof.

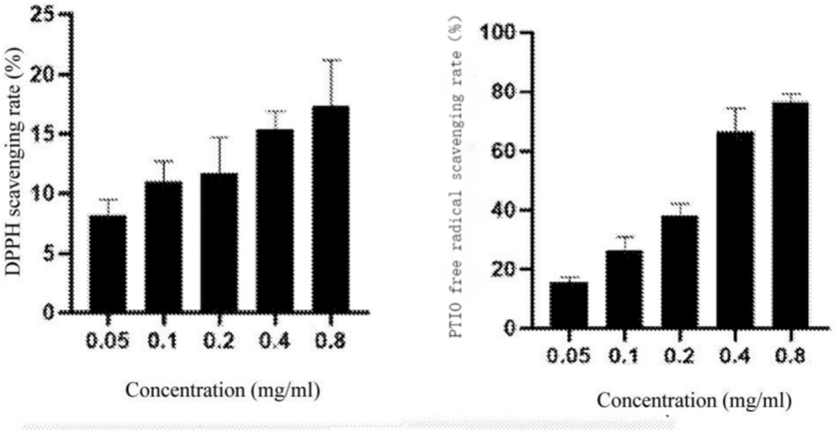


Fig. 1

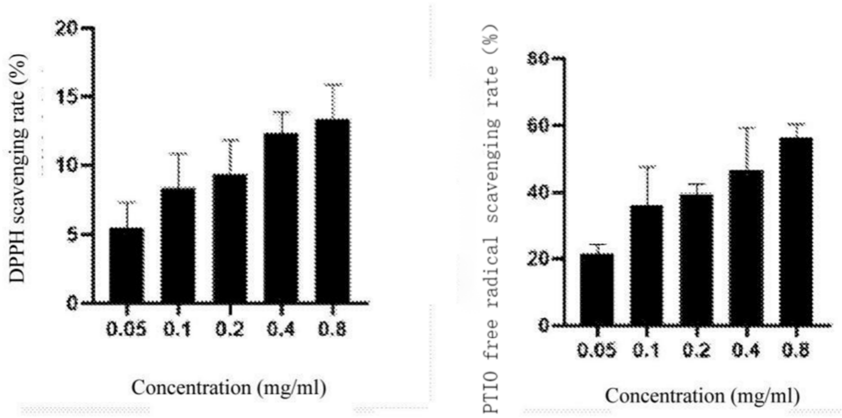


Fig. 2

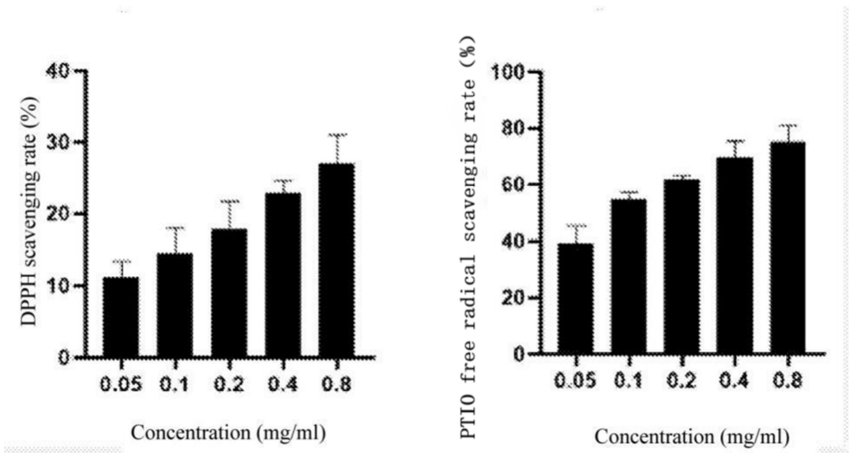


Fig. 3

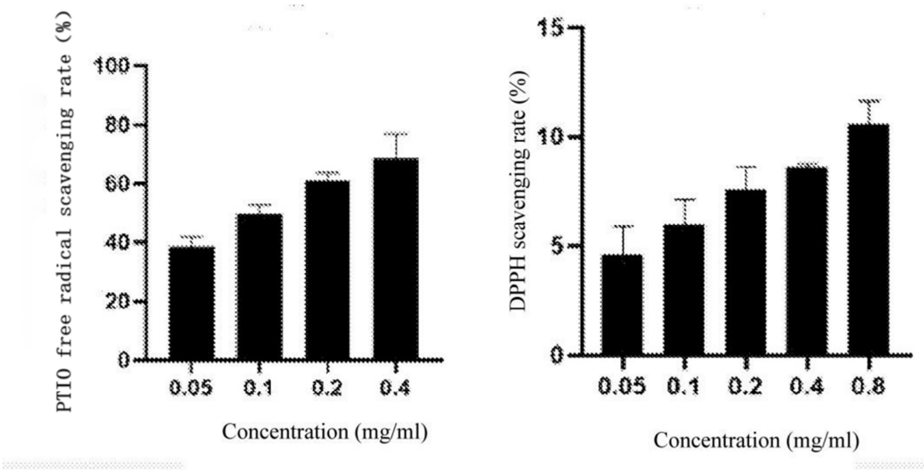


Fig. 4

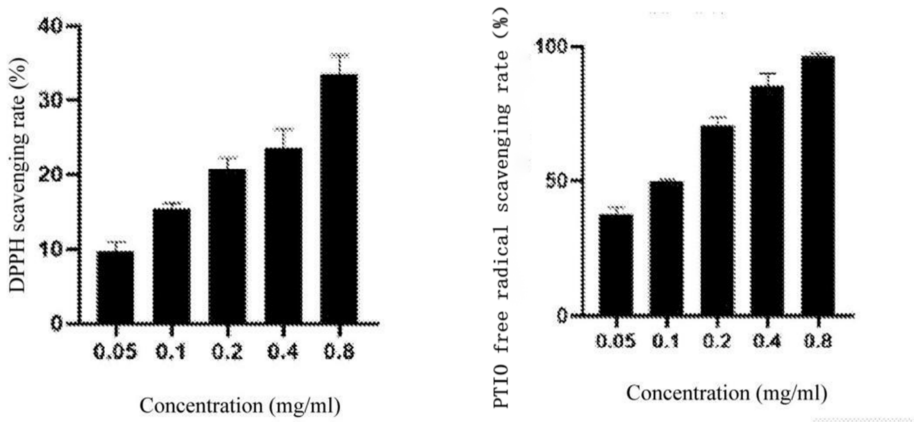


Fig. 5

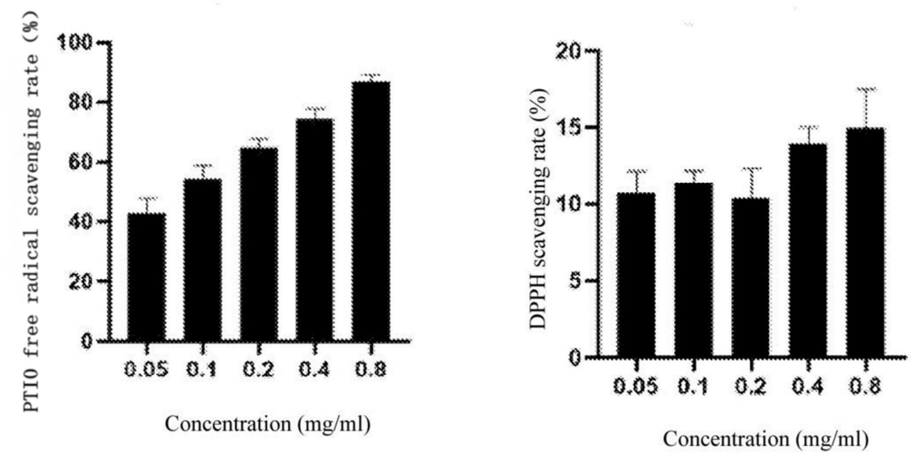


Fig. 6

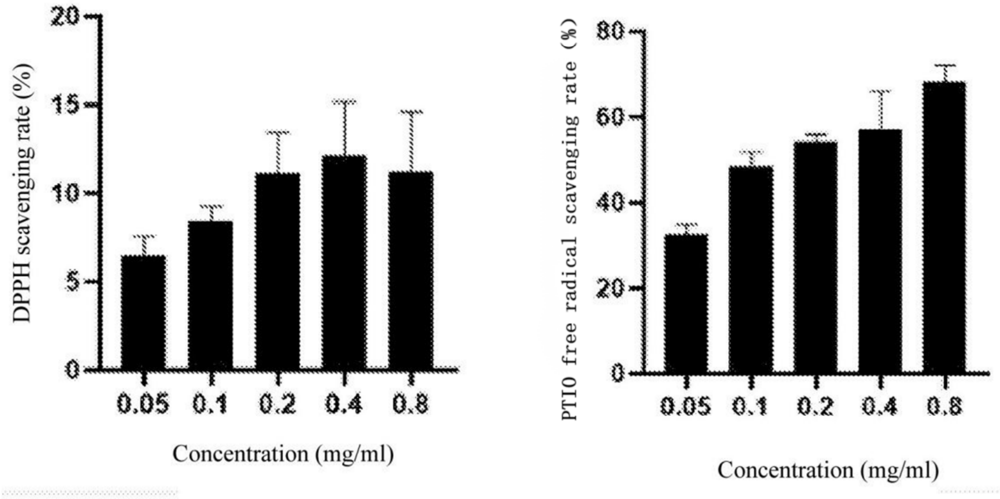


Fig. 7

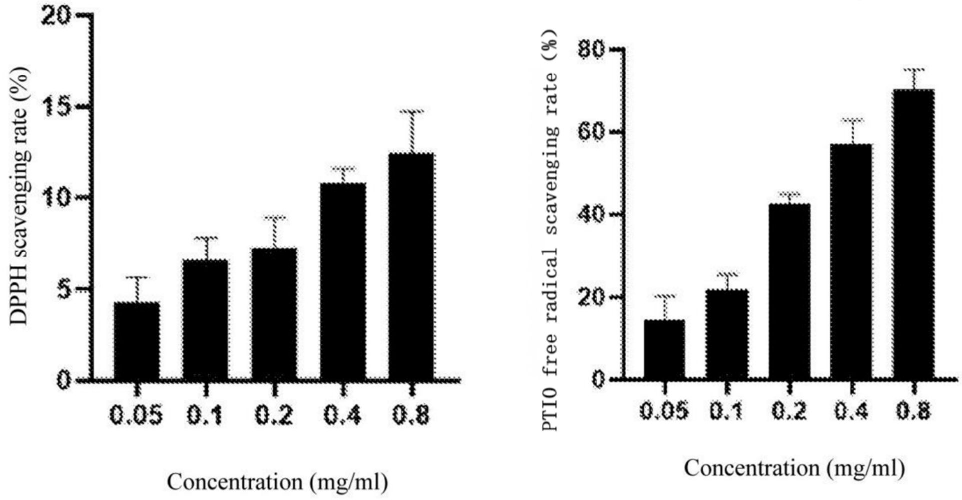


Fig. 8

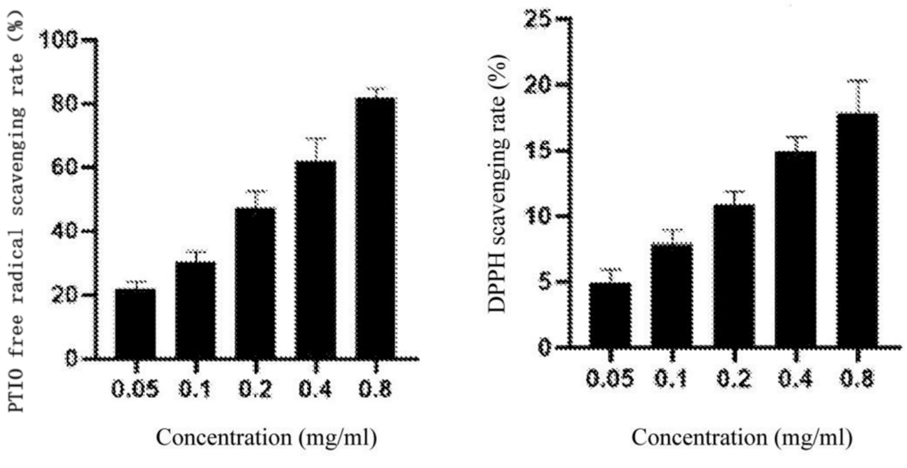


Fig. 9

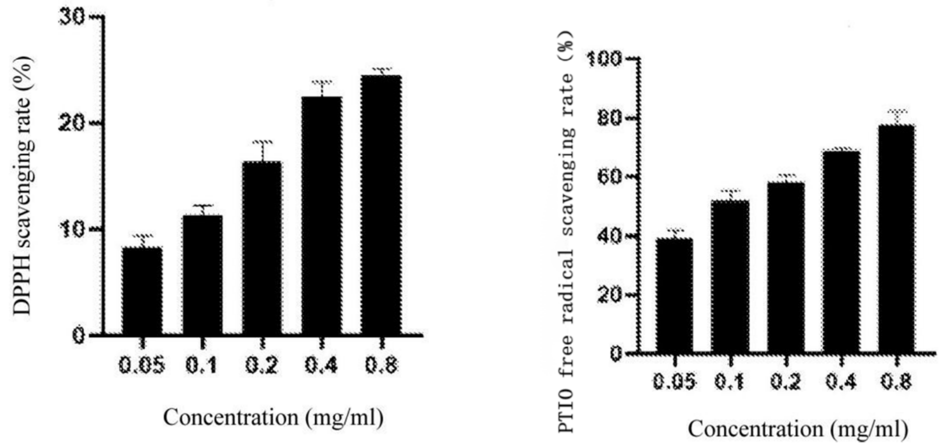


Fig. 10

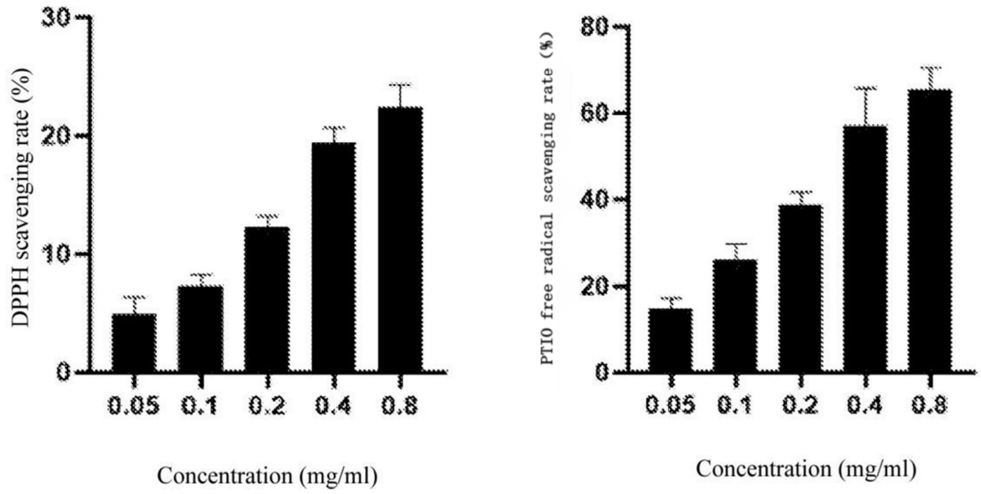


Fig. 11

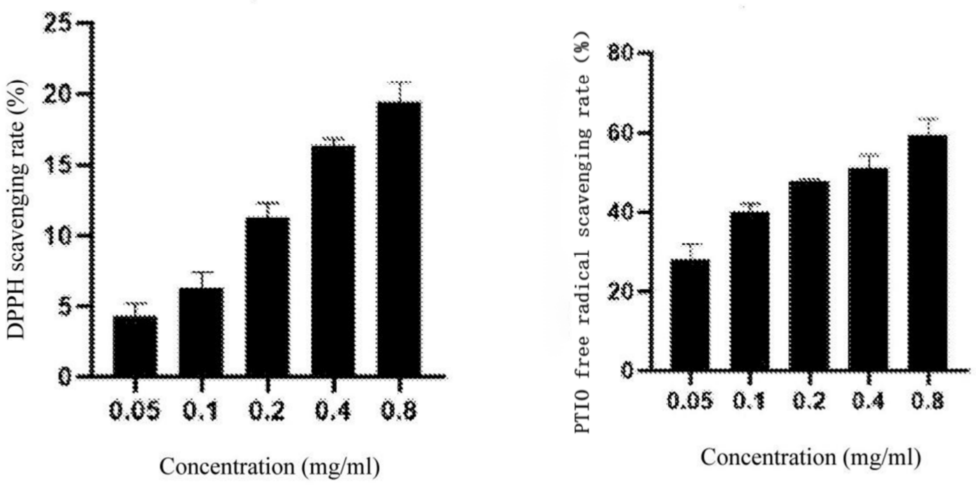


Fig. 12

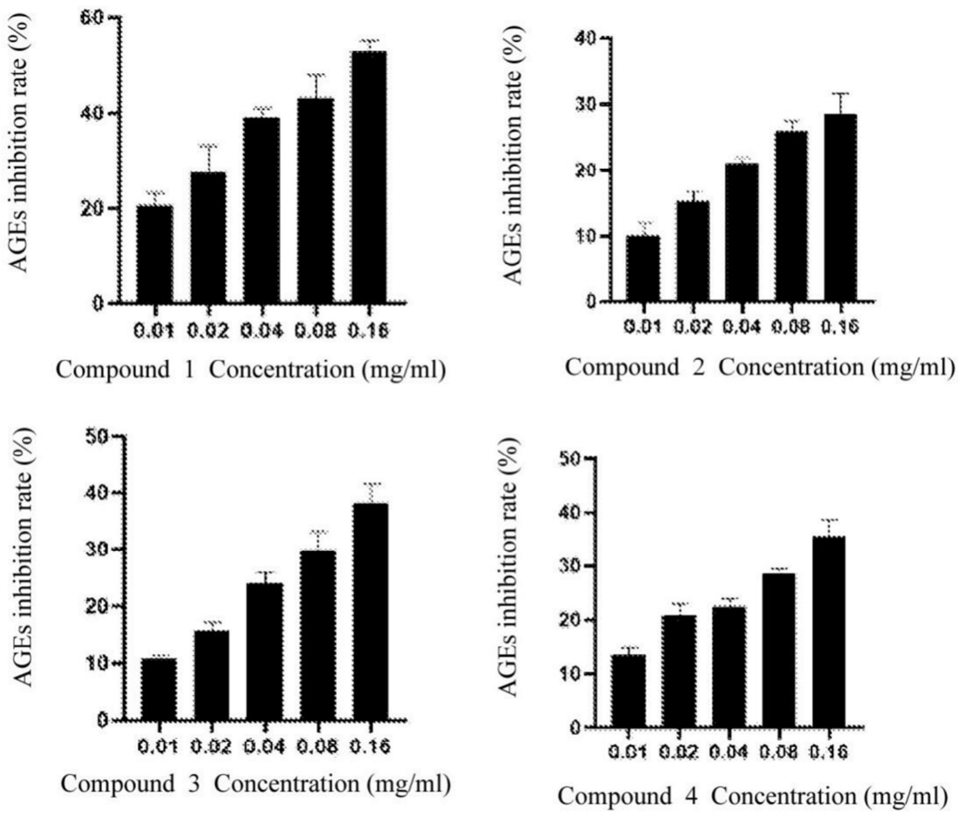
AGEs inhibition rate (%)
0
20
40
60
0.01 0.02 0.04 0.08 0.16
Compound 1 Concentration (mg/ml)
AGEs inhibition rate (%)
0
10
20
30
40
0.01 0.02 0.04 0.08 0.16
Compound 2 Concentration (mg/ml)
AGEs inhibition rate (%)
0
10
20
30
40
50
0.01 0.02 0.04 0.08 0.16
Compound 3 Concentration (mg/ml)
AGEs inhibition rate (%)
0
10
20
30
40
50
0.01 0.02 0.04 0.08 0.16
Compound 4 Concentration (mg/ml)

Fig. 13

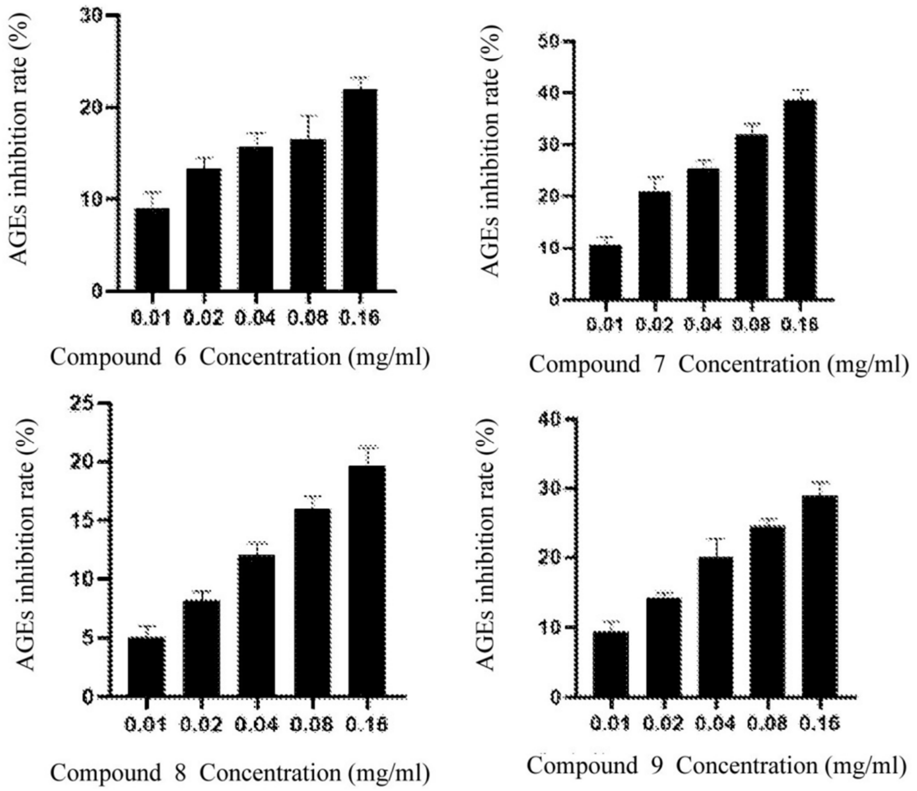
AGEs inhibition rate (%)
0
10
20
30
0.01 0.02 0.04 0.08 0.16
Compound 6 Concentration (mg/ml)
AGEs inhibition rate (%)
0
10
20
30
40
50
0.01 0.02 0.04 0.08 0.16
Compound 7 Concentration (mg/ml)
AGEs inhibition rate (%)
0
5
10
15
20
25
0.01 0.02 0.04 0.08 0.16
Compound 8 Concentration (mg/ml)
AGEs inhibition rate (%)
0
10
20
30
40
0.01 0.02 0.04 0.08 0.16
Compound 9 Concentration (mg/ml)

Fig. 14

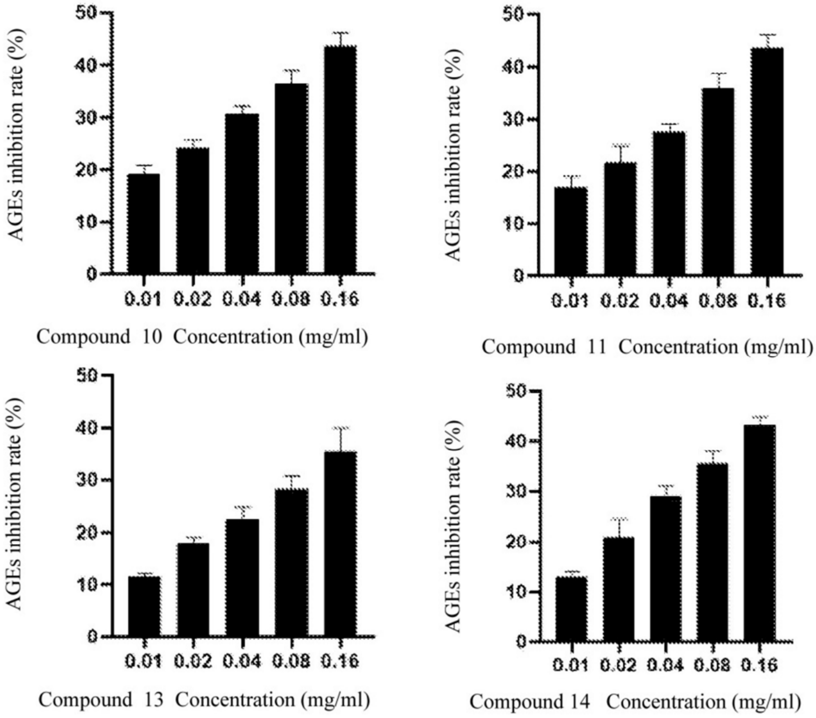
AGEs inhibition rate (%)
0
10
20
30
40
50
0.01 0.02 0.04 0.08 0.16
Compound 10 Concentration (mg/ml)
Compound 11 Concentration (mg/ml)
Compound 13 Concentration (mg/ml)
Compound 14 Concentration (mg/ml)

Fig. 15

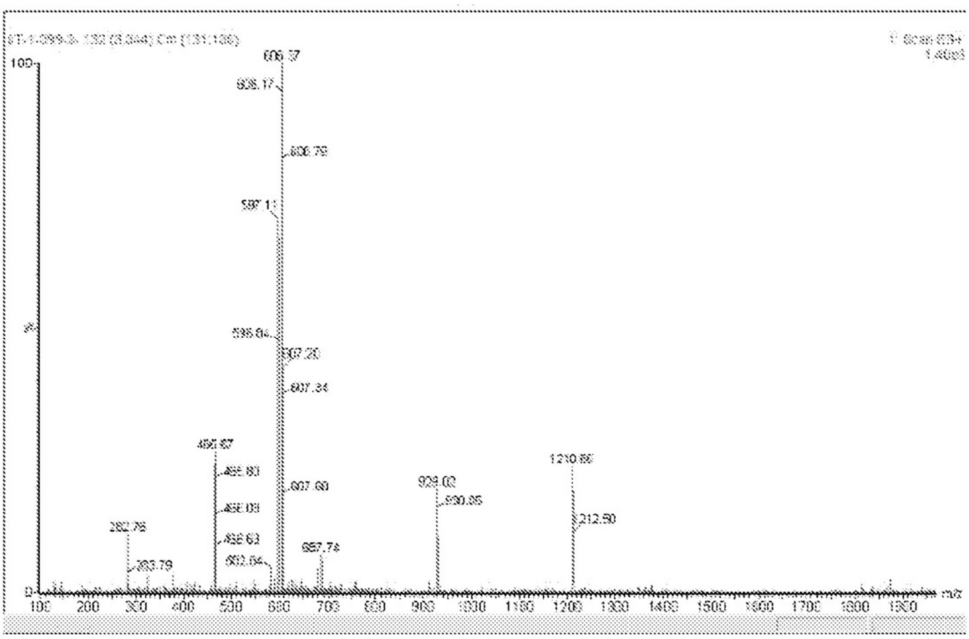

Fig. 16

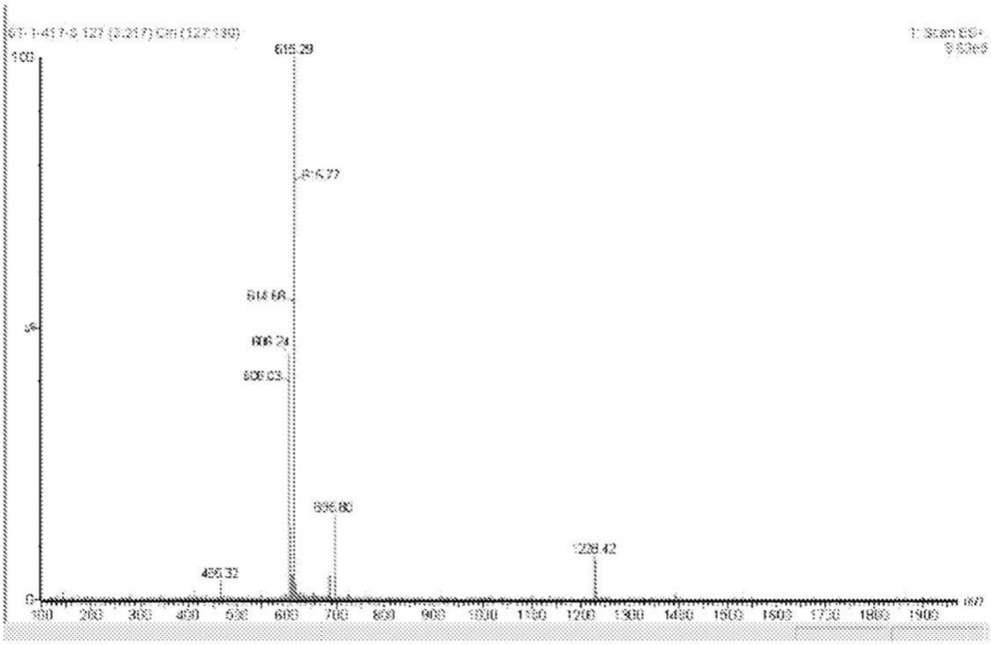
1: Scan ES+
615.29
616.27
614.68
606.24
608.03
696.80
1228.42
100
%
0
100
200
300
400
500
600
700
800
900
1000
1100
1200
1300
1400
1500
1600
1700
1800
1900

Fig. 17

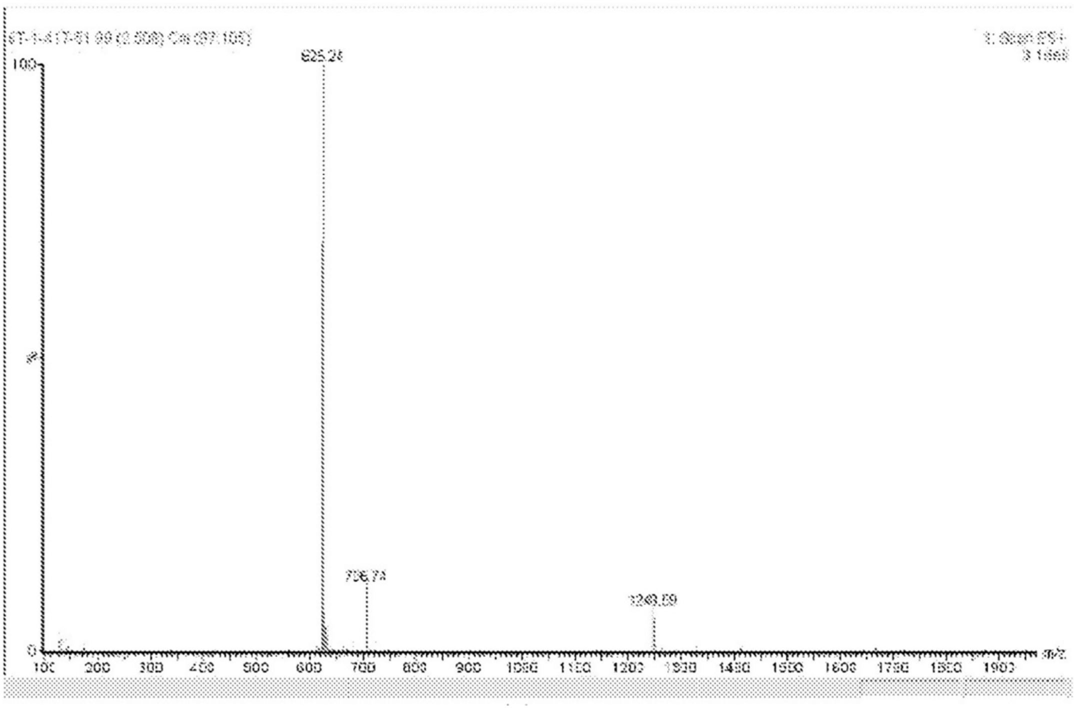
1: Scan ES+
625.24
706.74
1248.59
100
%
0
100
200
300
400
500
600
700
800
900
1000
1100
1200
1300
1400
1500
1600
1700
1800
1900

Fig. 18

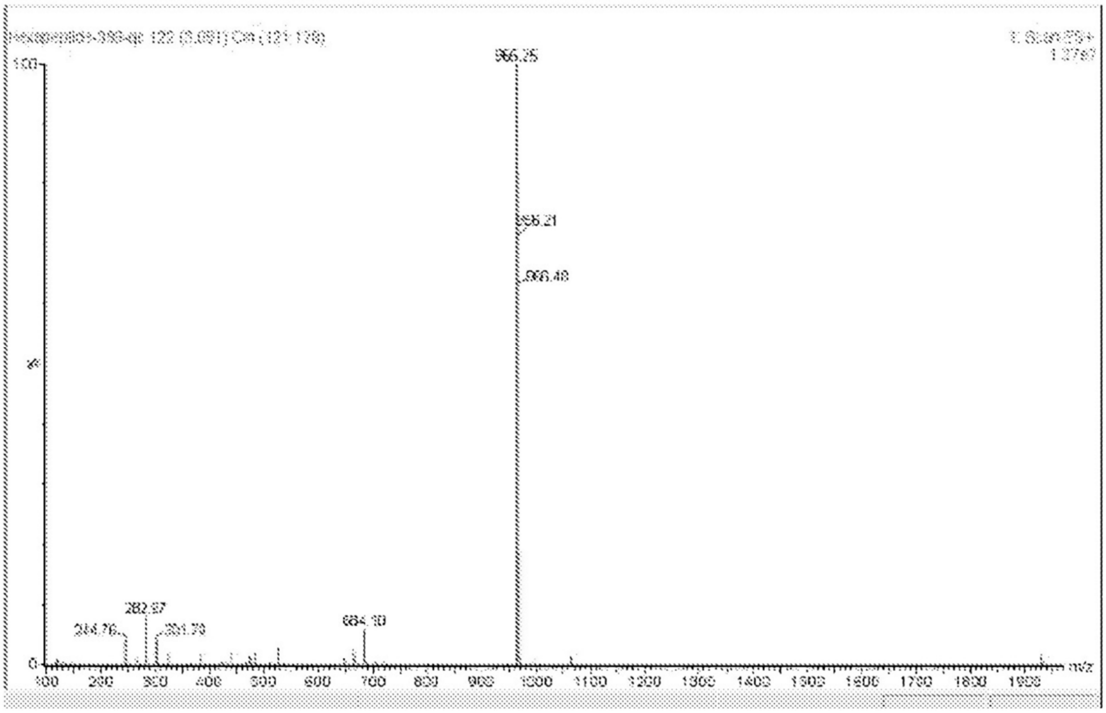
966.25
966.21
966.48
%
282.97
244.76
301.79
684.10
m/z

Fig. 19

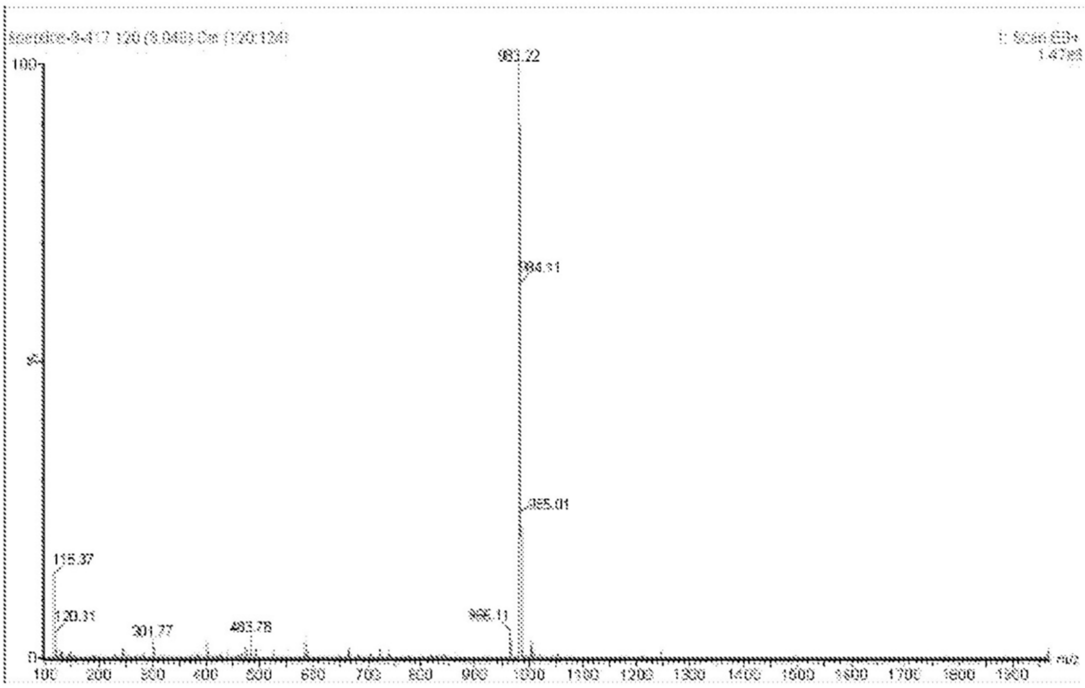
983.22
984.11
%
985.01
115.37
120.31
301.77
483.78
966.11
m/z

Fig. 20

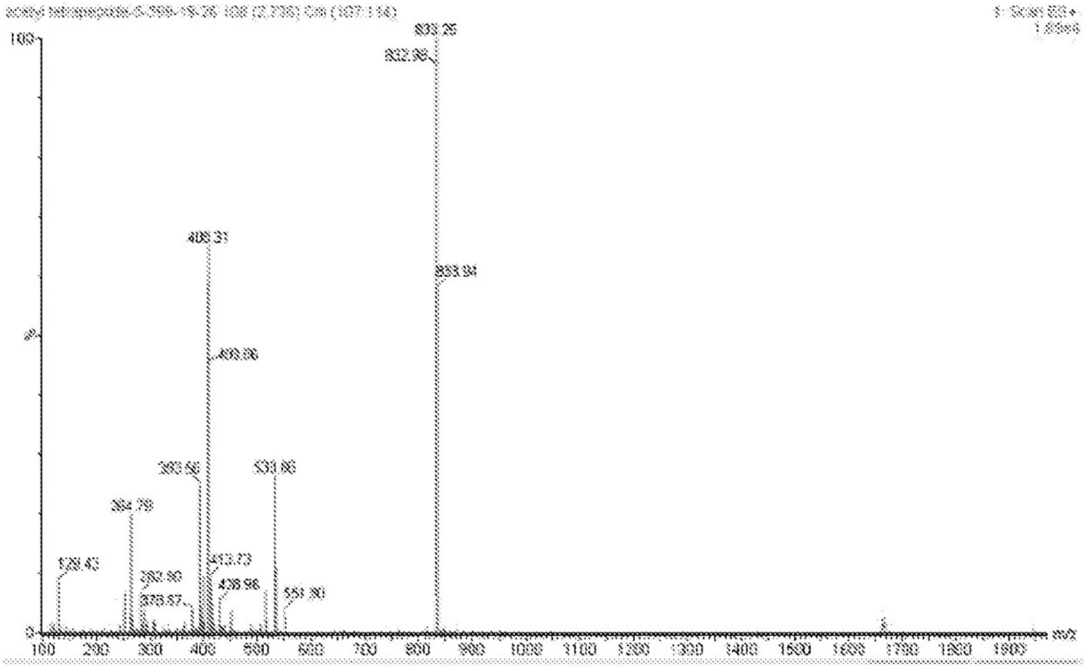
833.26
832.98
833.94
408.31
409.06
393.56
533.66
264.79
128.43
413.73
282.90
438.98
551.80
378.57
100
%
0
100
200
300
400
500
600
700
800
900
1000
1100
1200
1300
1400
1500
1600
1700
1800
1900

Fig. 21

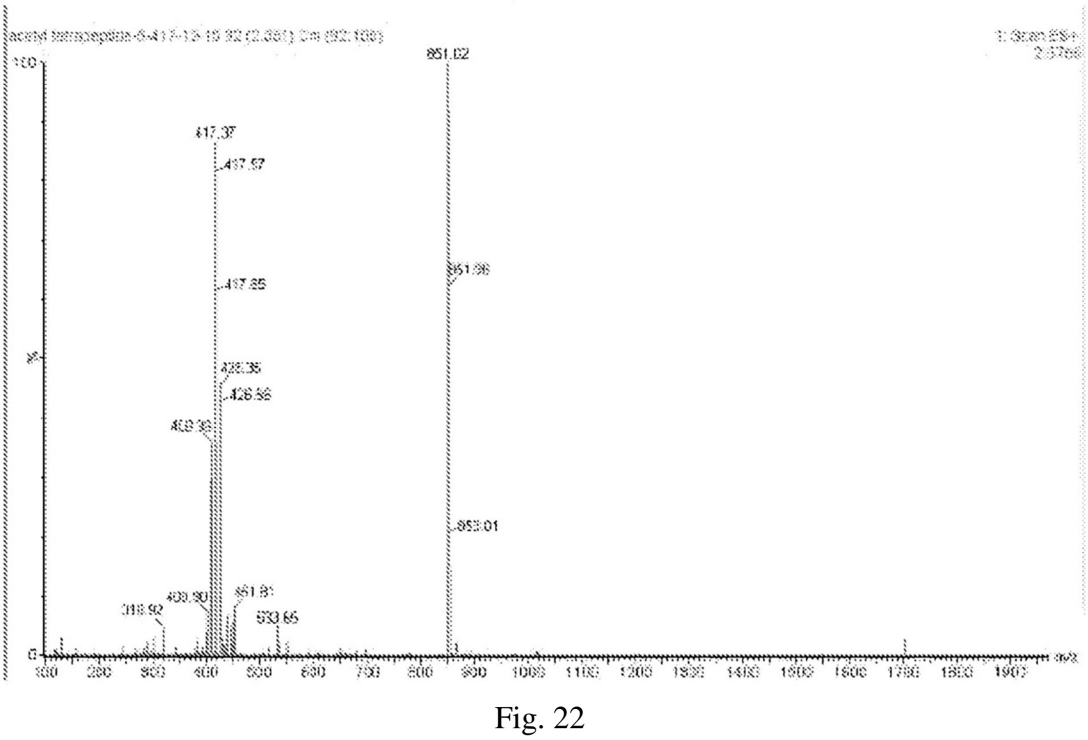
851.02
417.37
417.57
851.96
417.85
426.35
426.56
409.30
853.01
318.92
400.90
451.81
533.65
100
%
0
100
200
300
400
500
600
700
800
900
1000
1100
1200
1300
1400
1500
1600
1700
1800
1900

Fig. 22

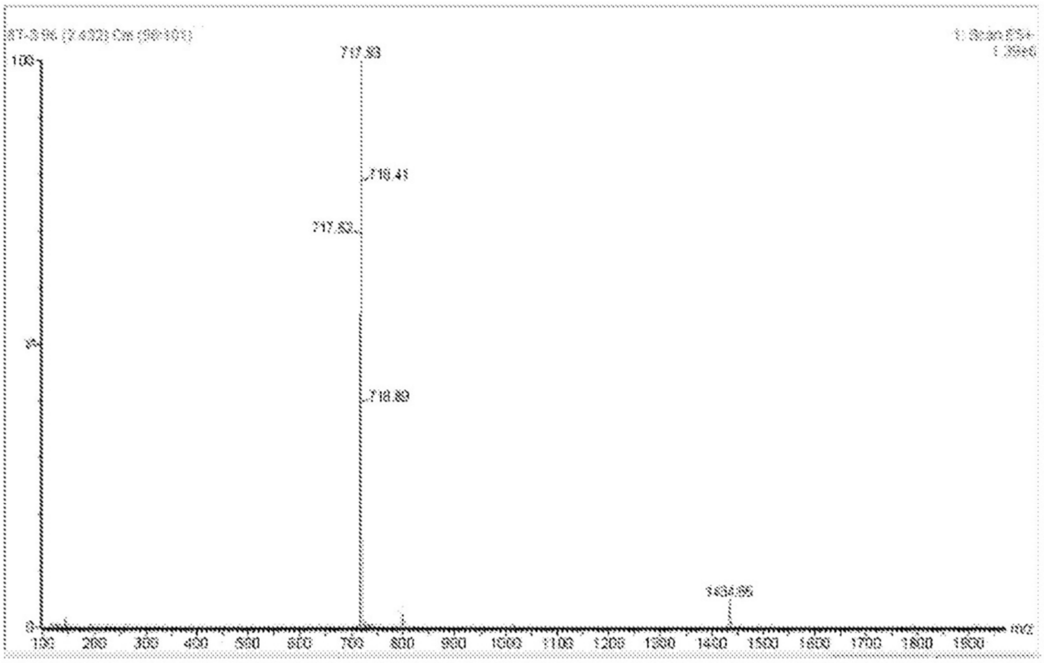

Fig. 23

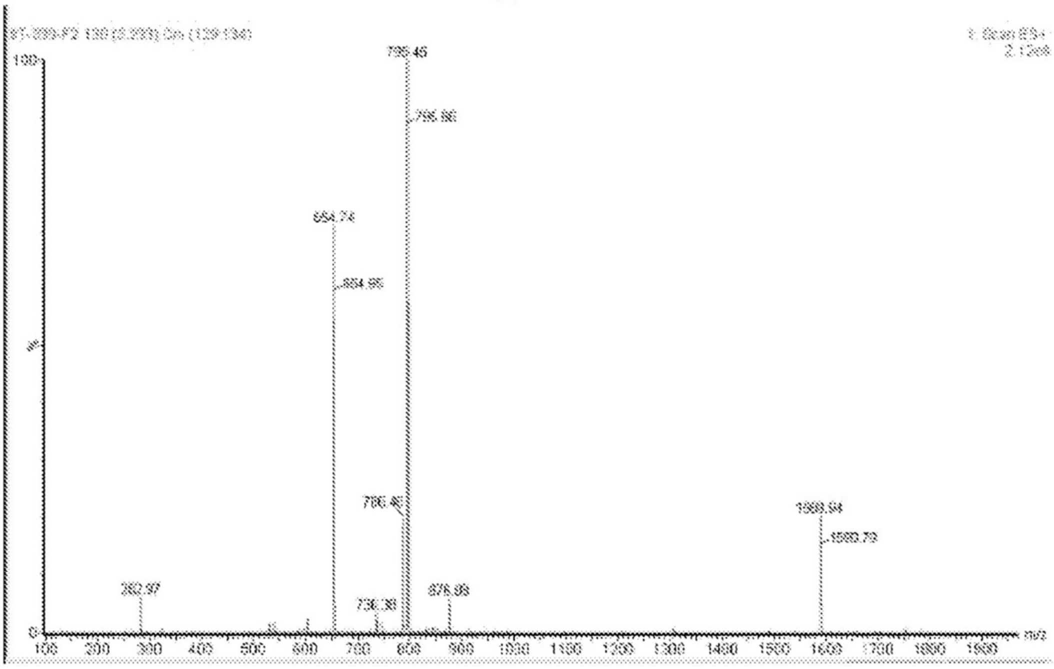

Fig. 24

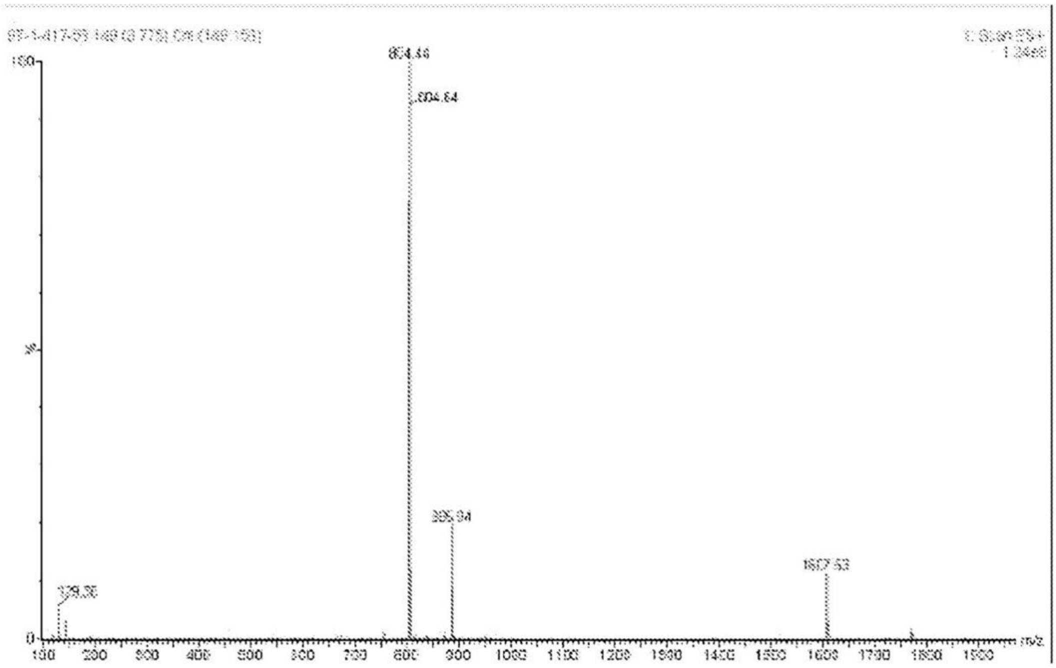

Fig. 25

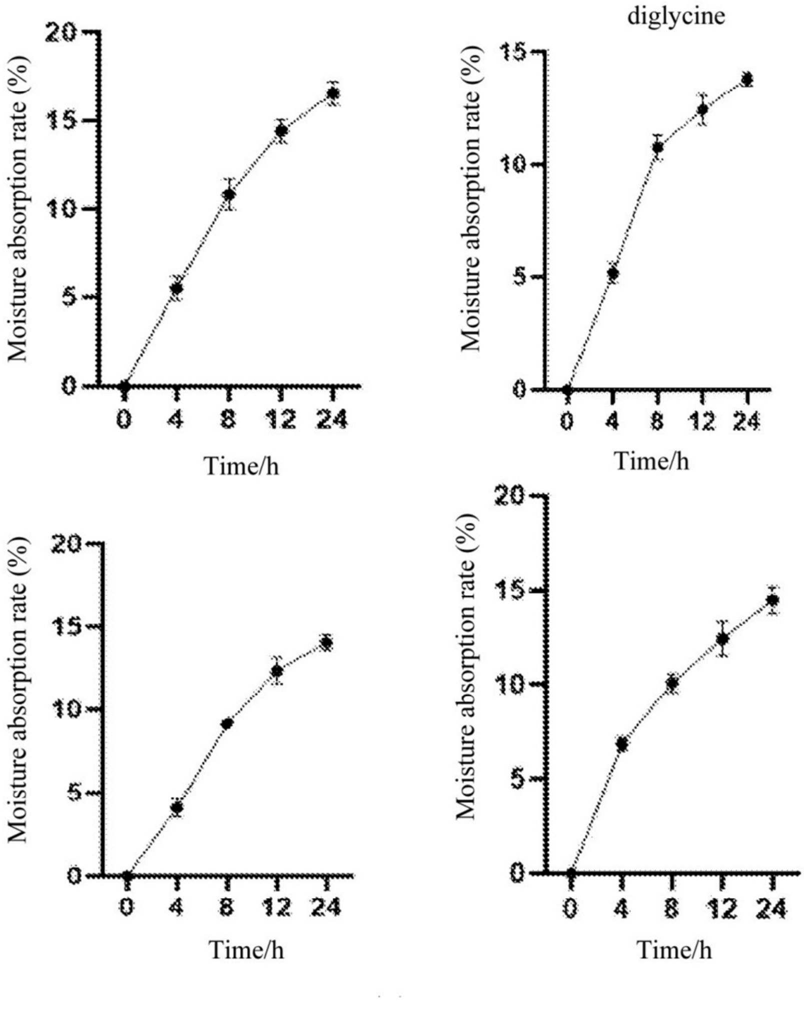


Fig. 26

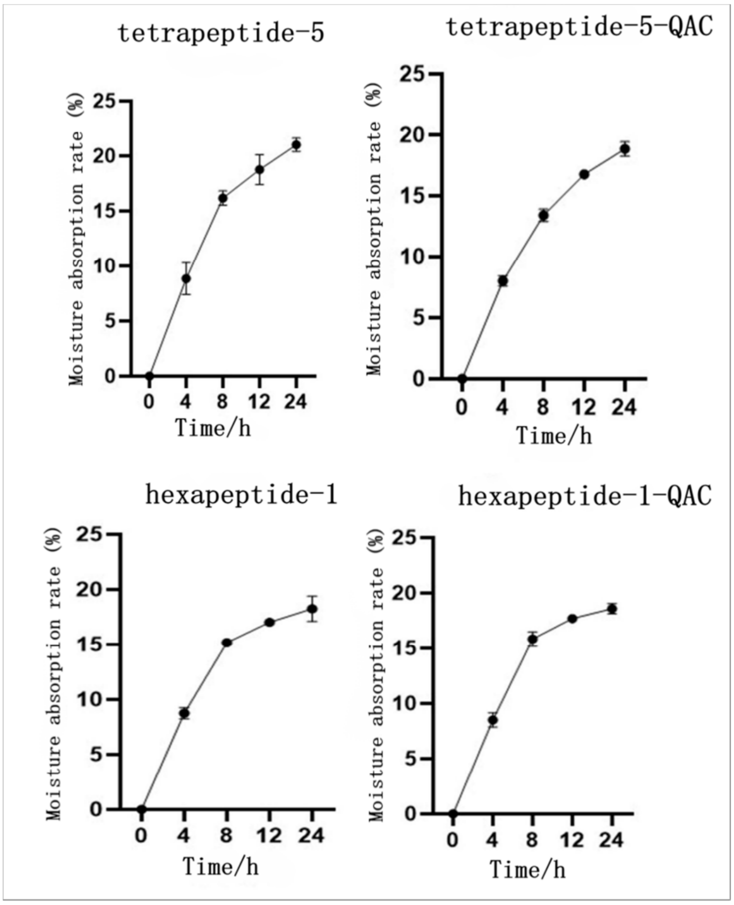
tetrapeptide-5
tetrapeptide-5-QAC
hexapeptide-1
hexapeptide-1-QAC
Moisture absorption rate (%)
Time/h
0
5
10
15
20
25
0
4
8
12
24

Fig. 27

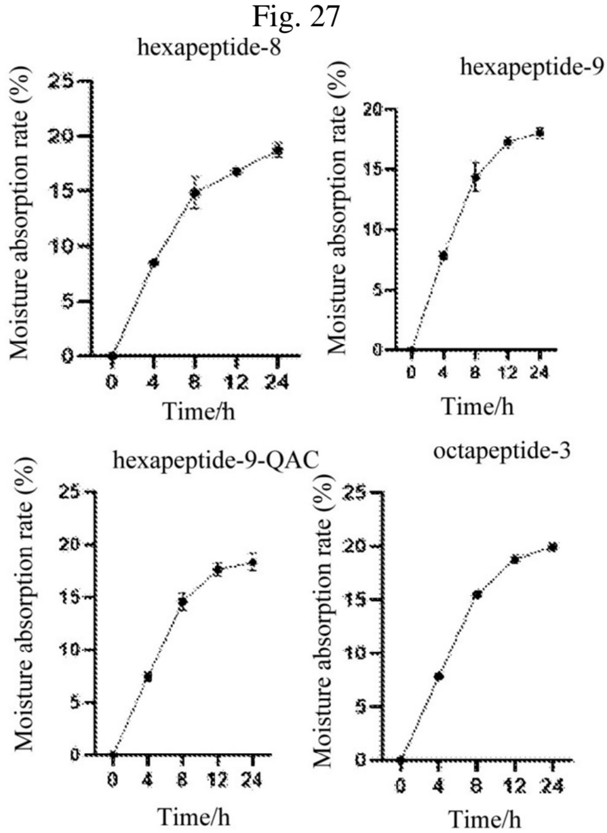
hexapeptide-8
hexapeptide-9
hexapeptide-9-QAC
octapeptide-3
Moisture absorption rate (%)
Time/h
0
5
10
15
20
25
0
4
8
12
24

Fig. 28

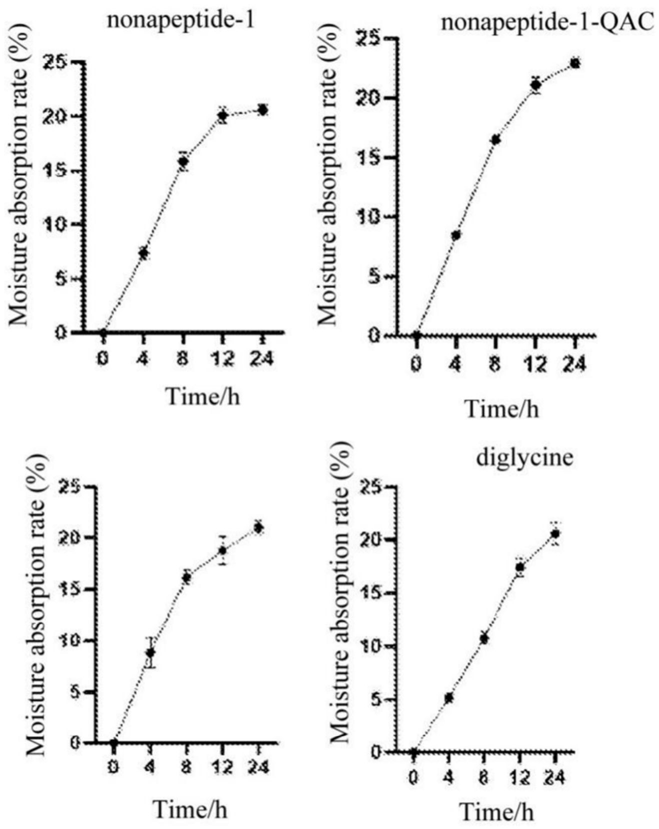
nonapeptide-1
nonapeptide-1-QAC
diglycine
Moisture absorption rate (%)
Time/h
0
5
10
15
20
25
0
4
8
12
24

Fig. 29

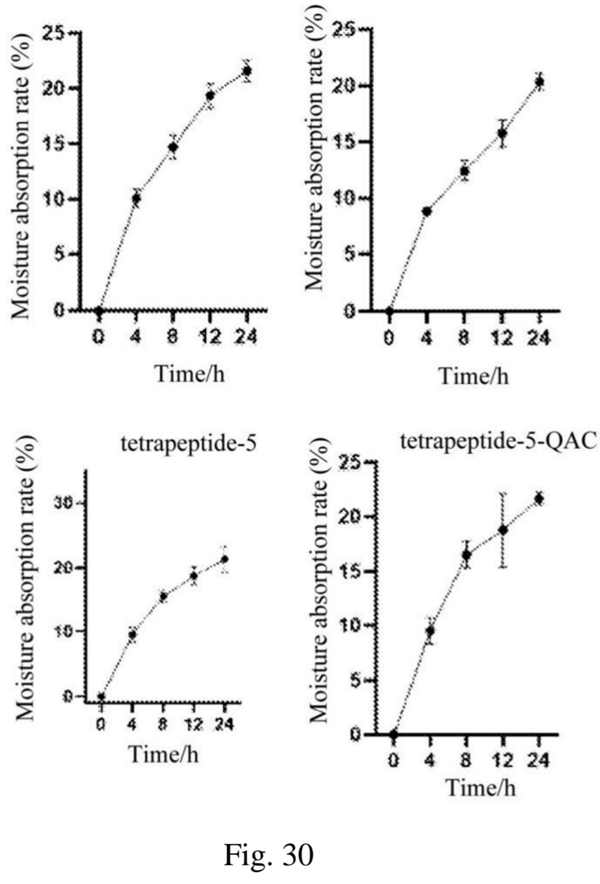
tetrapeptide-5
tetrapeptide-5-QAC
Moisture absorption rate (%)
Time/h
0
5
10
15
20
25
30
0
4
8
12
24

Fig. 30

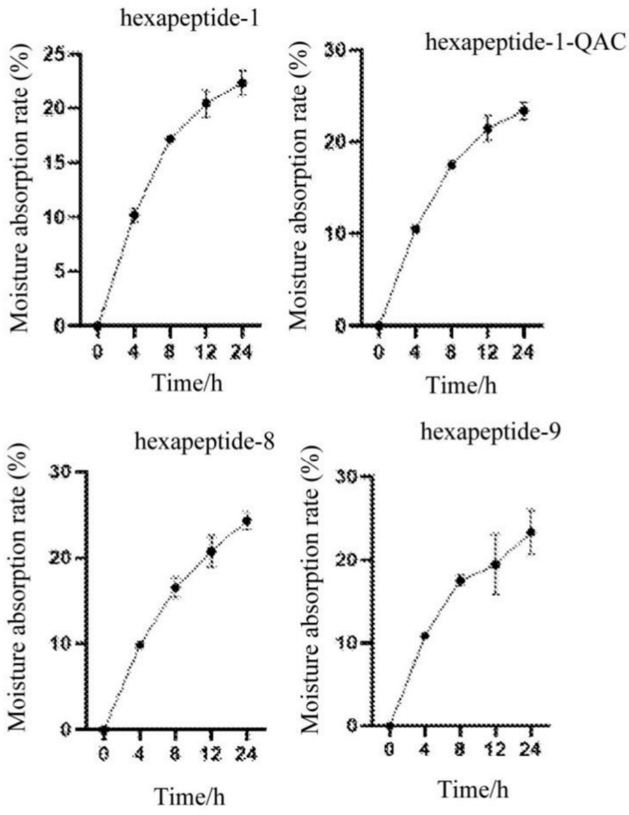
hexapeptide-1
Moisture absorption rate (%)
0
5
10
15
20
25
0 4 8 12 24
Time/h
hexapeptide-1-QAC
Moisture absorption rate (%)
0
10
20
30
0 4 8 12 24
Time/h
hexapeptide-8
Moisture absorption rate (%)
0
10
20
30
0 4 8 12 24
Time/h
hexapeptide-9
Moisture absorption rate (%)
0
10
20
30
0 4 8 12 24
Time/h

Fig. 31

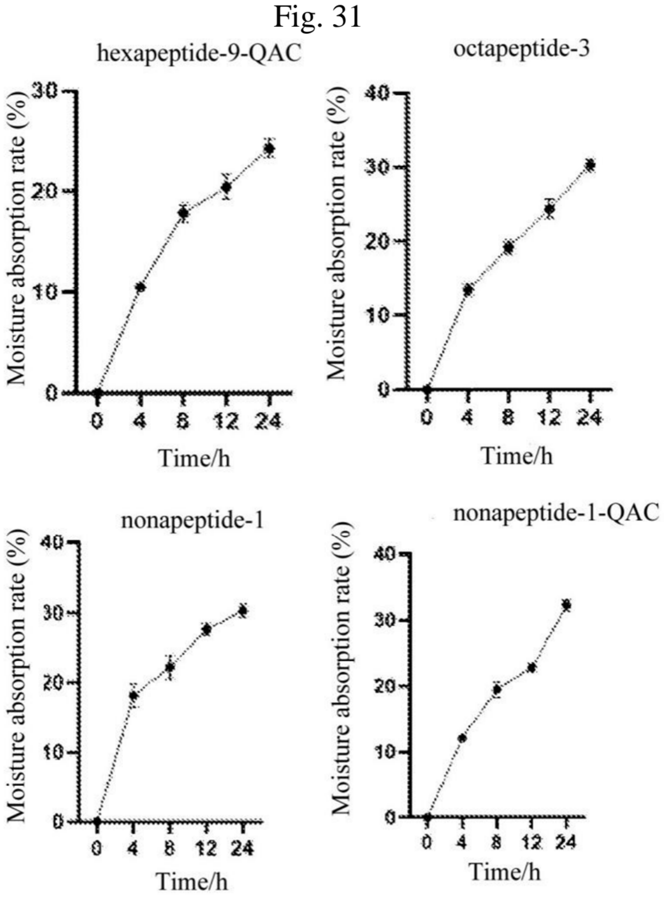
hexapeptide-9-QAC
Moisture absorption rate (%)
0
10
20
30
0 4 8 12 24
Time/h
octapeptide-3
Moisture absorption rate (%)
0
10
20
30
40
0 4 8 12 24
Time/h
nonapeptide-1
Moisture absorption rate (%)
0
10
20
30
40
0 4 8 12 24
Time/h
nonapeptide-1-QAC
Moisture absorption rate (%)
0
10
20
30
40
0 4 8 12 24
Time/h

Fig. 32

**INTERNATIONAL SEARCH REPORT**

International application No. **PCT/CN2024/073623**

**A. CLASSIFICATION OF SUBJECT MATTER**

C07K5/062(2006.01)i；C07K5/103(2006.01)i；C07K7/06(2006.01)i；C07K1/06(2006.01)i

According to International Patent Classification (IPC) or to both national classification and IPC

**B. FIELDS SEARCHED**

Minimum documentation searched (classification system followed by classification symbols)

IPC：C07K5/-; C07K7/-; C07K1/-

Documentation searched other than minimum documentation to the extent that such documents are included in the fields searched

Electronic data base consulted during the international search (name of data base and, where practicable, search terms used)

CNTXT, CNKI, VEN, ENTXTC, ISI_Web of Science: 氨基酸, 丙氨酸, 多肽, 多肽缩合, 鞘氨醇碱, 神经酰胺, 缩合, 衍生, 酰胺, ceramide, fmoc, amino acid, sphingosine base

**C. DOCUMENTS CONSIDERED TO BE RELEVANT**

| Category* | Citation of document, with indication, where appropriate, of the relevant passages | Relevant to claim No. |
|---|---|---|
| PX | CN 116496345 A (SHENZHEN DIKEMAN BIOTECHNOLOGY CO., LTD.) 28 July 2023 (2023-07-28)<br>entire document | 1-14 |
| PX | CN 116444393 A (SHENZHEN DIKEMAN BIOTECHNOLOGY CO., LTD.) 18 July 2023 (2023-07-18)<br>entire document | 1-14 |
| A | CN 115260091 A (SHENZHEN DIKEMAN BIOTECHNOLOGY CO., LTD.) 01 November 2022 (2022-11-01)<br>entire document | 1-14 |
| A | CN 115433100 A (SHENZHEN DIKEMAN BIOTECHNOLOGY CO., LTD.) 06 December 2022 (2022-12-06)<br>entire document | 1-14 |
| A | US 2010197954 A1 (YANG HAO et al.) 05 August 2010 (2010-08-05)<br>entire document | 1-14 |
| A | CN 105765072 A (KAO CORP.) 13 July 2016 (2016-07-13)<br>entire document | 1-14 |

☐ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **15 March 2024** | **22 March 2024** |
| Name and mailing address of the ISA/CN<br>**China National Intellectual Property Administration (ISA/CN)**<br>**China No. 6, Xitucheng Road, Jimenqiao, Haidian District, Beijing 100088** | Authorized officer<br><br>Telephone No. |

Form PCT/ISA/210 (second sheet) (July 2022)

**INTERNATIONAL SEARCH REPORT**
**Information on patent family members**

International application No.
**PCT/CN2024/073623**

| Patent document cited in search report | Publication date (day/month/year) | Patent family member(s) | Publication date (day/month/year) |
|---|---|---|---|
| CN 116496345 A | 28 July 2023 | None | |
| CN 116444393 A | 18 July 2023 | None | |
| CN 115260091 A | 01 November 2022 | None | |
| CN 115433100 A | 06 December 2022 | None | |
| US 2010197954 A1 | 05 August 2010 | WO 2008030840 A2 | 13 March 2008 |
| | | WO 2008030840 A3 | 24 July 2008 |
| | | US 7960589 B2 | 14 June 2011 |
| CN 105765072 A | 13 July 2016 | US 2016298150 A1 | 13 October 2016 |
| | | US 10066249 B2 | 04 September 2018 |
| | | EP 3071700 A1 | 28 September 2016 |
| | | EP 3071700 B1 | 20 July 2022 |
| | | KR 20160086846 A | 20 July 2016 |
| | | WO 2015076423 A1 | 28 May 2015 |
| | | JP 2015119698 A | 02 July 2015 |
| | | JP 6389400 B2 | 12 September 2018 |

Form PCT/ISA/210 (patent family annex) (July 2022)